# EUROPEAN PATENT APPLICATION

(11) **EP 4 197 561 A1**
(43) Date of publication of application: **21.06.2023**
(21) Application number: 21214757.3
(22) Date of filing: 15.12.2021
(51) Int. Cl.: A61K 51/08, A61P 35/00

(54) **UROKINASE-TYPE PLASMINOGEN ACTIVATOR RECEPTOR (UPAR)-PET/CT IN NEUROENDOCRINE NEOPLAMS**

(71) Applicant: Curasight A/S, 2200 Copenhagen N (DK)
(72) Inventor: Kjær, Andreas, 2100 Copenhagen Ø (DK)
(74) Representative: Plougmann Vingtoft a/s

(57) **Abstract**

The present invention relates to positron-emitting imaging agents for use in the prognosis of a neuroendocrine neoplasm (NEN) patient in by PET imaging of the cancer, wherein said imaging agent comprises a uPAR binding peptide coupled via the chelating agent NOTA or DOTA to the radionuclide 68Ga or 64Cu. The invention also relates to compositions comprising a radiopharmaceutical for use in the treatment or alleviation of a neuroendocrine neoplasm (NEN) in a subject, wherein said radiopharmaceutical comprises a radionuclide and a uPAR binding peptide.

## Description

### Technical field of the invention

The present invention relates to a positron-emitting imaging agent for use in the prognostication of a neuroendocrine neoplasm (NEN) patient by PET imaging of the cancer, wherein said imaging agent comprises a uPAR binding peptide coupled via the chelating agent NOTA or DOTA to the radionuclide 68Ga or 64Cu. The invention also relates to compositions comprising a radiopharmaceutical for use in the treatment or alleviation of a neuroendocrine neoplasm (NEN) in a subject, wherein said radiopharmaceutical comprises a radionuclide and a uPAR binding peptide. Finally, the invention related to the use of said imaging agents are used as companion diagnostic for the radiopharmaceutical for therapy.

### Background of the invention

Neuroendocrine neoplasms (NENs) originate from the neuroendocrine cells and are found primarily in the gastro-intestinal tract, pancreas and lungs. The clinical course for patients diagnosed with NEN ranges from indolent to highly aggressive. Origin of primary tumor, presence of metastases as well as tumor morphology and proliferation activity (i.e. Ki-67) are known prognostic factors. Patients are stratified by the WHO classification into NET G1 (Ki67 <3%), NET G2 (Ki67 3-20%), NET G3 (Ki67 > 20% and well differentiated) and NEC (Ki67 > 20% and poorly differentiated) (1). To further improve prognostication and guide decisions on treatment, non-invasive monitoring of tumor markers may be useful. PET is ideally suited for this task, as different specific radiotracers may be applied to visualize whole-body expression of tumor markers non-invasively. In NEN particularly, radiotracers for somatostatin receptor expression (e.g. ⁶⁴Cu-DOTATATE or ⁶⁸Ga-DOTATATE) and glucose metabolism (¹⁸F-FDG) are useful for diagnosis, prognostication and therapy selection (2,3). In addition, peptide receptor radionuclide therapy (PRRT) with e.g. ¹⁷⁷Lu-DOTATATE targeting somatostatin receptors is approved for low grade NEN, whereas lower somatostatin receptor expression in high grade NEN can limit its application.

Hence, improved prognostic tools in relation to NEN would be advantageous, and in particular a more efficient and/or reliable treatments of subtypes of NEN would be advantageous.

### Summary of the invention

Urokinase plasminogen activator receptor (uPAR) is a promising diagnostic and prognostic biomarker as well as a target for therapy that has been extensively investigated in several cancer entities (4) but never in relation to NEN. uPAR is anchored to the cell membrane on the surface and localizes the proteolytic activity of its ligand, urokinase plasminogen activator (uPA). In normal tissues, uPAR expression is limited, however in cancer, uPAR expression is upregulated. Apart from uPA, uPAR also interacts with other proteins, among others the integrin family of membrane proteins. Collectively, uPAR is involved in promoting cell proliferation, motility, invasion, proteolysis and angiogenesis (4-6). Due to its integral role in cancer, our group has developed the PET radiotracer ⁶⁸Ga-NOTA-AE105 employing a high-affinity antagonist for uPAR (7-9). Safety and biodistribution were investigated in a phase I study, also showing accumulation of ⁶⁸Ga-NOTA-AE105 in primary tumors and metastases, as well as correlation with uPAR expression in excised tumor samples (7). Recently, we reported that ⁶⁸Ga-NOTA-AE105 uPAR PET is able to discriminate between low-risk and intermediate risk profiles in prostate cancer (10). Furthermore, we have previously shown a high efficacy of uPAR targeted PRRT in preclinical trials in prostate and colorectal cancers (*11*,*12)*. Thus, uPAR being a marker of aggressive disease may show upregulation in high grade NEN, and could provide a target for PRRT in these patients.

The aim of this study of patients with NEN was to assess tumor uptake and clinical outcome using uPAR-PET (exemplified by ⁶⁸Ga-NOTA-AE105 PET/CT). We hypothesized that uPAR-PET/CT with ⁶⁸Ga-NOTA-AE105 would show accumulation in NEN and that the uptake of the uPAR tracer would be associated with progression-free survival (PFS) and overall survival (OS) (see example 5).

Surprisingly, it was found that uPAR-PET was a stronger prognostication tool (higher Hazard Ratios (HR)) (Figure 4A-4B) than commonly used PET modalities for neuroendocrine tumors such as FDG-PET (See Figure 6 - reference figure) and somatostatin receptor imaging, e.g. 64Cu-DOTATATE (see Figure 7 - reference figure). The latter was not significant regarding OS.

Thus, an object of the present invention relates to the provision of improved prognostic tools for NEN.

In particular, it is also an object of the present invention to provide improved treatment protocols for patients suffering from NEN.

Thus, one aspect of the invention relates to a positron-emitting imaging agent for use in the prognostication of a neuroendocrine neoplasm (NEN) patient by PET imaging of the cancer,
wherein said imaging agent comprises a uPAR binding peptide coupled via the chelating agent NOTA or DOTA to the radionuclide 68Ga or 64Cu; and
wherein the uPAR binding peptide is (D-Asp)-([beta]-cyclohexyl-L-alanine)-(Phe)-(D-Ser)-(D-Arg)-(Tyr)-(Leu)-(Trp)-(Ser) or a uPAR binding variant thereof.

The two uPAR-PET tracers 64Cu-DOTA-AE105 and ⁶⁸Ga-NOTA-AE105 share the same binding moiety, the peptide AE105. In addition, they have both been tested in both humans and animals (see ref (7), (28) and WO2014/086364 A1) and have demonstrated similar uptake in breast, bladder and prostate cancer. Accordingly, without being bound by theory, it is believed PET tracers comprising
- 64Cu or 68Ga as radionuclide;
- DOTA or NOTA as chelator; and
- the uPAR binding peptide according to the present invention;
will work in a similar manner also for NEN patients.

Another aspect of the invention relates to a positron-emitting imaging agent for use in the prognostication of a neuroendocrine neoplasm (NEN) patient by PET imaging of the cancer,
wherein said imaging agent comprises a uPAR binding peptide coupled via the chelating agent NOTA to the radionuclide 68Ga; and
wherein the uPAR binding peptide is (D-Asp)-([beta]-cyclohexyl-L-alanine)-(Phe)-(D-Ser)-(D-Arg)-(Tyr)-(Leu)-(Trp)-(Ser) (AE105) or uPAR binding variants thereof. Such imaging agent has been tested in the example section.

Yet another aspect of the present invention is to provide a method of in vivo imaging by PET imaging, for assessing the prognosis of a Neuroendocrine neoplasm (NEN) in a patient, said method comprising:
a) provision of a subject to which a imaging agent according to the invention has been previously administered;
b) detecting by in vivo PET imaging the radioactive emissions from the radioisotope of the administered imaging agent of step a);
c) generating an image representative of the location and/or amount of said radioactive emissions;
d) determining the distribution and extent of uPAR expression in said subject, wherein said expression is correlated with said signals emitted by said in vivo imaging agent; and
e) comparing the determined distribution and extent of uPAR expression to a threshold level;
   ▪ wherein a SUVmax and/or SUVmean and/or TLR quantification level above a threshold level is indicative of a poor Relapse-free survival (RFS) prognosis and/or poor overall survival (OS) prognosis; and
   ▪ wherein a SUVmax and/or SUVmean and/or TLR quantification level equal to or below a threshold level is indicative of good Relapse-free survival (RFS) prognosis and/or good overall survival (OS) prognosis.

Still another aspect of the present invention is the use of a positron-emitting imaging agent according to the invention in the prognosis of neuroendocrine neoplasm (NEN) patients by in vivo PET imaging of uPAR expressing tumors,
wherein said imaging agent comprises a uPAR binding peptide coupled via the chelating agent NOTA or DOTA to the radionuclide 68Ga or 64Cu;
wherein the uPAR binding peptide is (D-Asp)-([beta]-cyclohexyl-L-alanine)-(Phe)-(D-Ser)-(D-Arg)-(Tyr)-(Leu)-(Trp)-(Ser) or a uPAR binding variant thereof.

Preferably said imaging agent comprises a uPAR binding peptide coupled via the chelating agent NOTA to the radionuclide 68Ga

As outlined above, the methods of the invention and the compositions for use according to the invention, may also find use as a companion diagnostic. Thus, a further aspect of the invention relates to a positron-emitting imaging agent for use as companion diagnostic of a neuroendocrine neoplasm (NEN) patient by PET imaging of the cancer,
wherein said imaging agent comprises a uPAR binding peptide coupled via the chelating agent NOTA or DOTA to the radionuclide 68Ga or 64Cu; and
wherein the uPAR binding peptide is (D-Asp)-([beta]-cyclohexyl-L-alanine)-(Phe)-(D-Ser)-(D-Arg)-(Tyr)-(Leu)-(Trp)-(Ser) or a uPAR binding variant thereof;
wherein
   ∘ a SUVmax and/or SUVmean and/or target-to liver ratio (TLR) quantification level above a threshold level is indicative of that a uPAR binding drug, such as the uPAR binding drug being a radiopharmaceutical, will be effective against said NEN; and
   ∘ a SUVmax and/or SUVmean and/or target-to liver ratio (TLR) quantification level equal to or below a threshold level is indicative of that a uPAR binding drug, such as the uPAR binding drug being a radiopharmaceutical, will not be effective against said NEN.

Yet an aspect of the invention relates to a composition comprising a radiopharmaceutical for use in the treatment or alleviation of a neuroendocrine neoplasm (NEN) in a subject
wherein said radiopharmaceutical comprises a radionuclide and a uPAR binding peptide; and
wherein the uPAR binding peptide is (D-Asp)-([beta]-cyclohexyl-L-alanine)-(Phe)-(D-Ser)-(D-Arg)-(Tyr)-(Leu)-(Trp)-(Ser) or a uPAR binding variant thereof; and
wherein said subject has been diagnosed with a uPAR expressing neuroendocrine neoplasm (NEN); and wherein said uPAR expression is above a predetermined threshold level,
wherein said threshold level is determined using a positron-emitting imaging agent according to the invention.

### Brief description of the figures

*Figure 1*

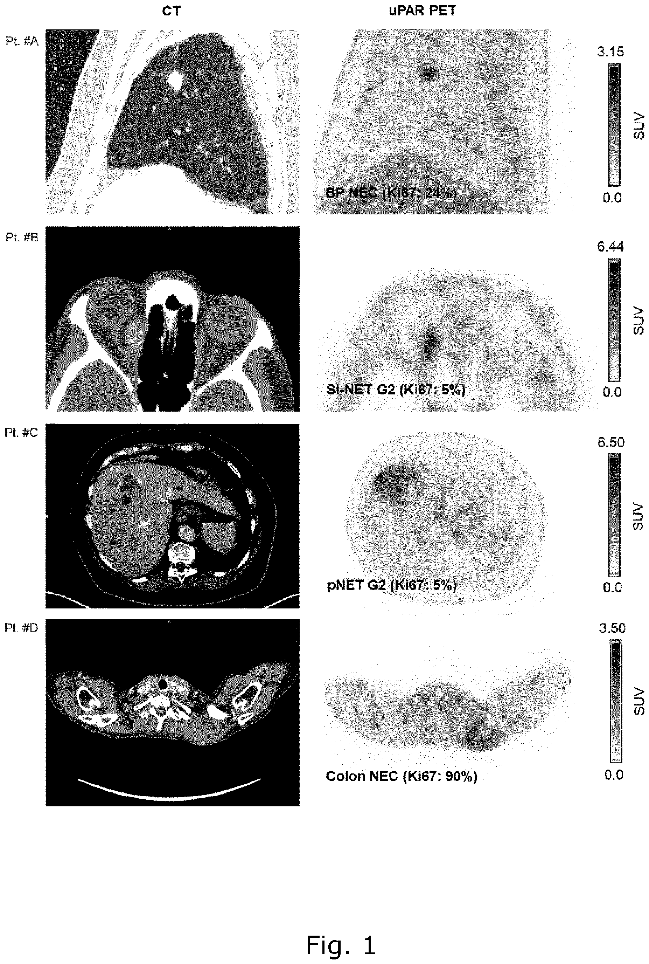

   Figure 1 shows representative examples of uPAR PET/CT imaging. **Left column:** CT. **Right column:** uPAR. 4 patients (#A-D) with high and low grade NEN were examined. The top of the individual scale bar corresponds to the SUVₘₐₓ value of the tumor. **Pt. #A:** Bronchopulmonary NEC (Ki67: 24%). **Pt. #B:** Orbital metastasis from small intestine NET G2 (Ki-67: 5%); **Pt. #C:** Large liver metastasis from pancreatic NET G2 (Ki-67: 5%). **Pt. #D:** Large intramuscular metastasis from primary colon NEC (Ki-67: 90%).
*Figure 2*

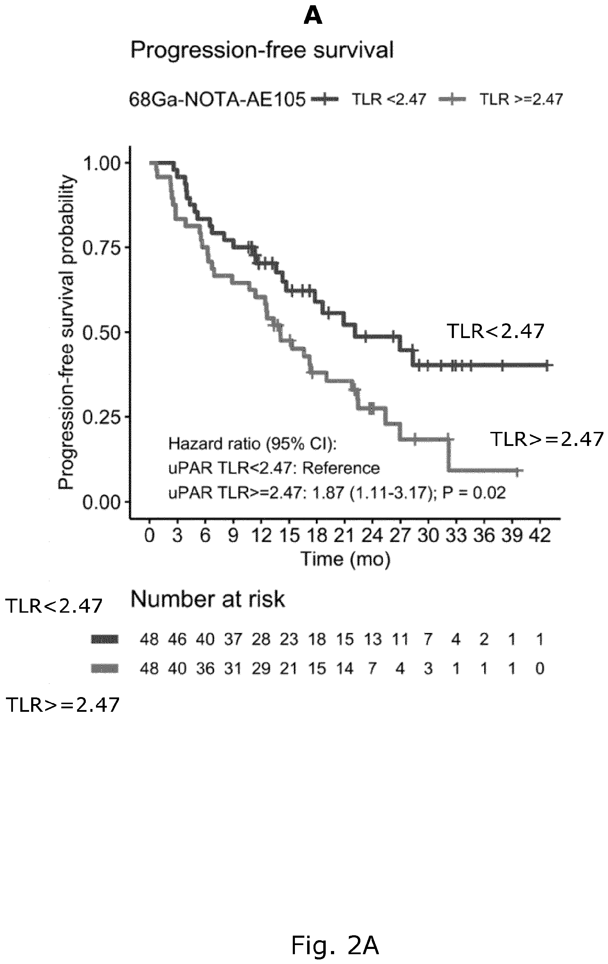

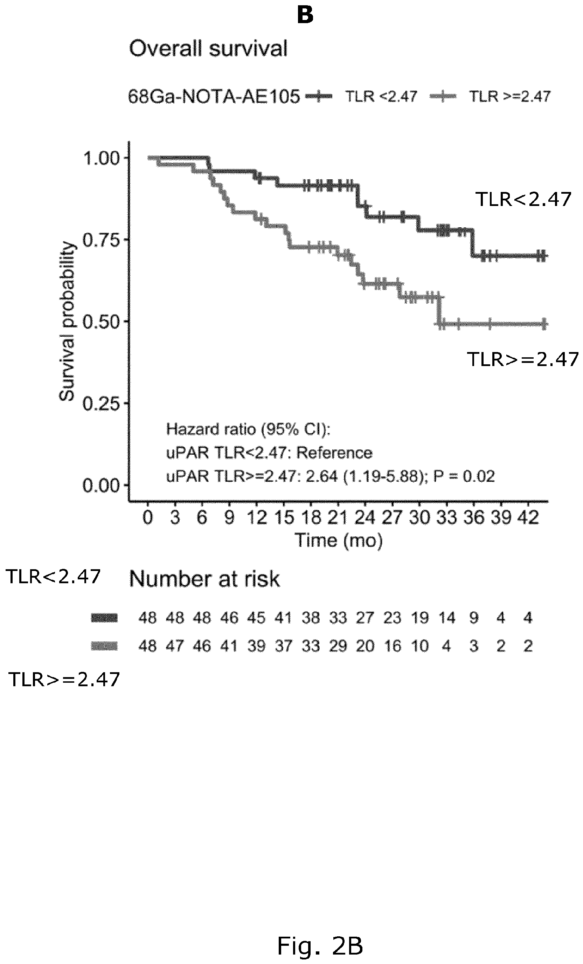

   Figure 2 shows Kaplan-Meier plots of **(A)** progression-free (PFS) survival and **(B)** overall survival using ⁶⁸Ga-NOTA-AE105 uPAR PET. uPAR target-to-liver-ratio dichotomized at median (TLR: 2.47).
*Figure 3*

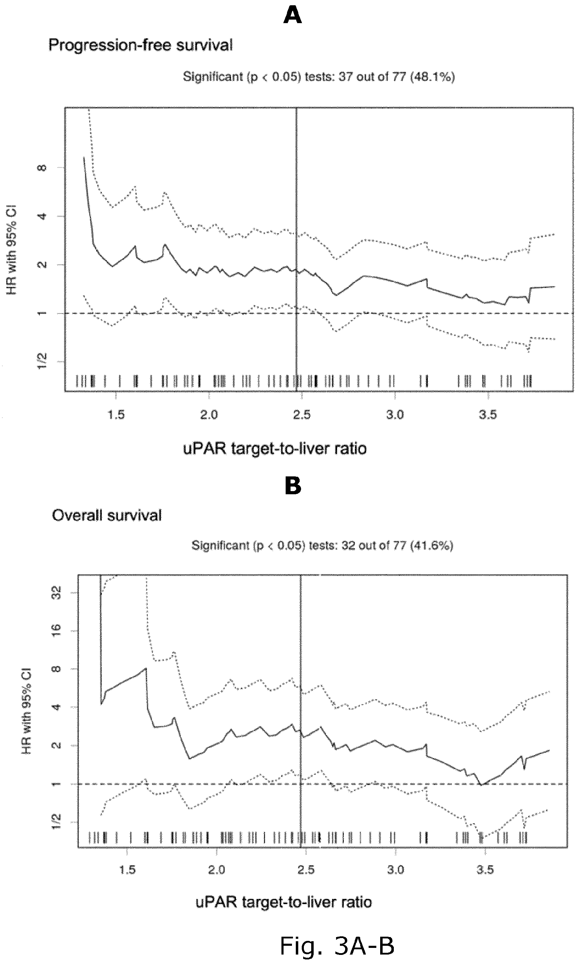

   Figure 3 shows "Cutoff Finder" output depicting hazard ratio and 95% confidence interval (HR with 95% CI) when uPAR TLR is dichotomized at any possible cutpoint (x-axis). **(A)** progression-free (PFS) survival and **(B)** overall survival using ⁶⁸Ga-NOTA-AE105 uPAR PET Solid vertical line is placed at median uPAR TLR (2.47).
*Figure 4*

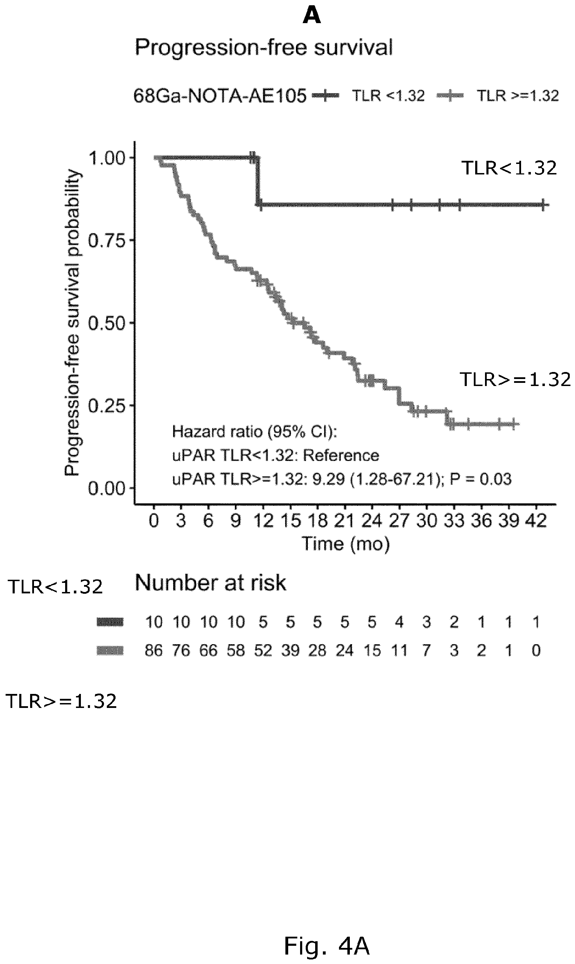

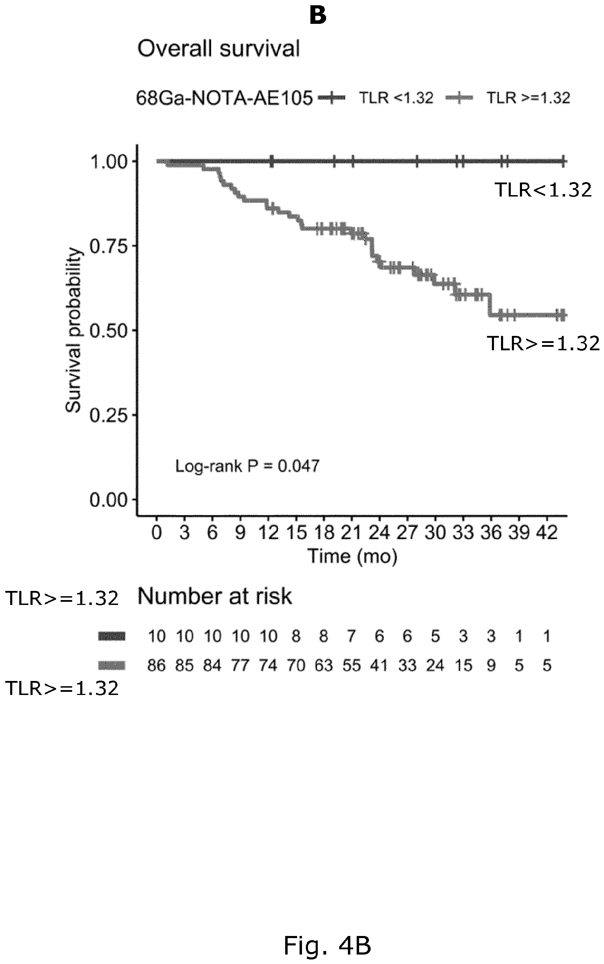

   Figure 4 shows Kaplan-Meier plots of **(A)** progression-free (PFS) survival and **(B)** overall survival using ⁶⁸Ga-NOTA-AE105 uPAR PET. uPAR TLR dichotomized at 1.32.
*Figure 5*

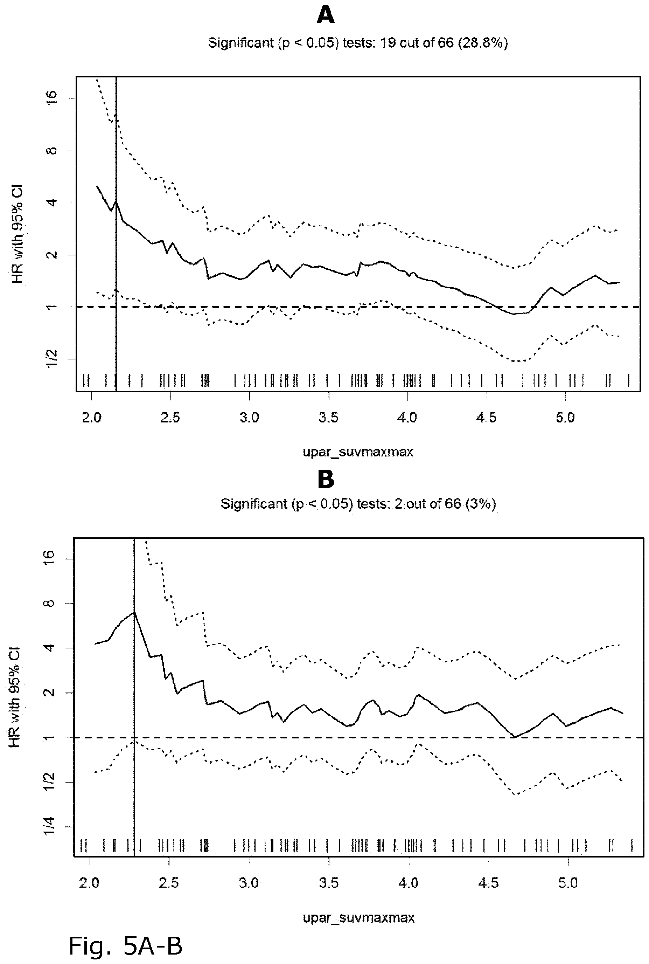

   Figure 5 shows Cut-off finder for SUVmax. **(A)** PFS and **(B)** OS.
*Figure 6*

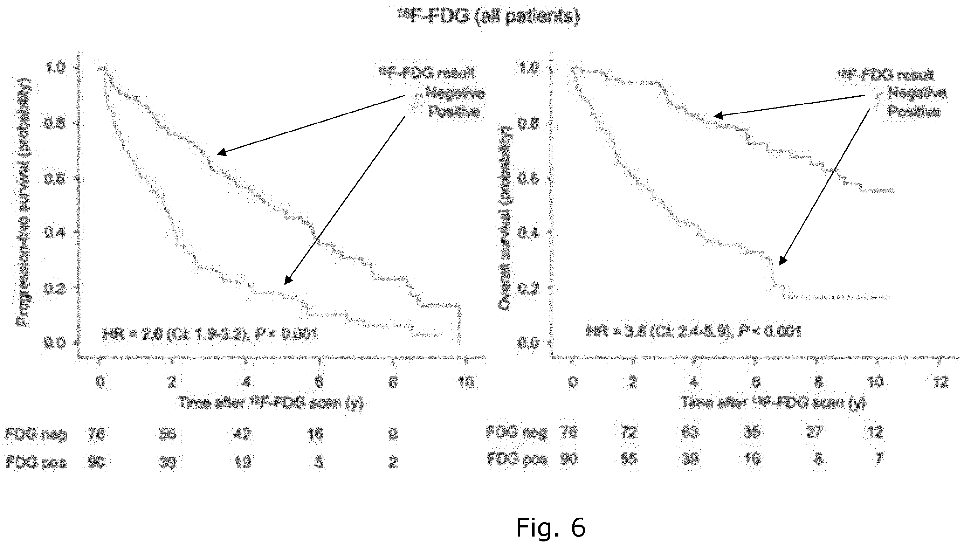

   Figure 6 shows Kaplan-Meier plots of FDG-PET and **(left)** progression free survival (PFS) and **(right)** overall survival (OS) in neuroendocrine tumors patients. From ref. 3 (Binderup et al. J Nucl Med 2021). Reference figure, not part of the invention.
*Figure 7*

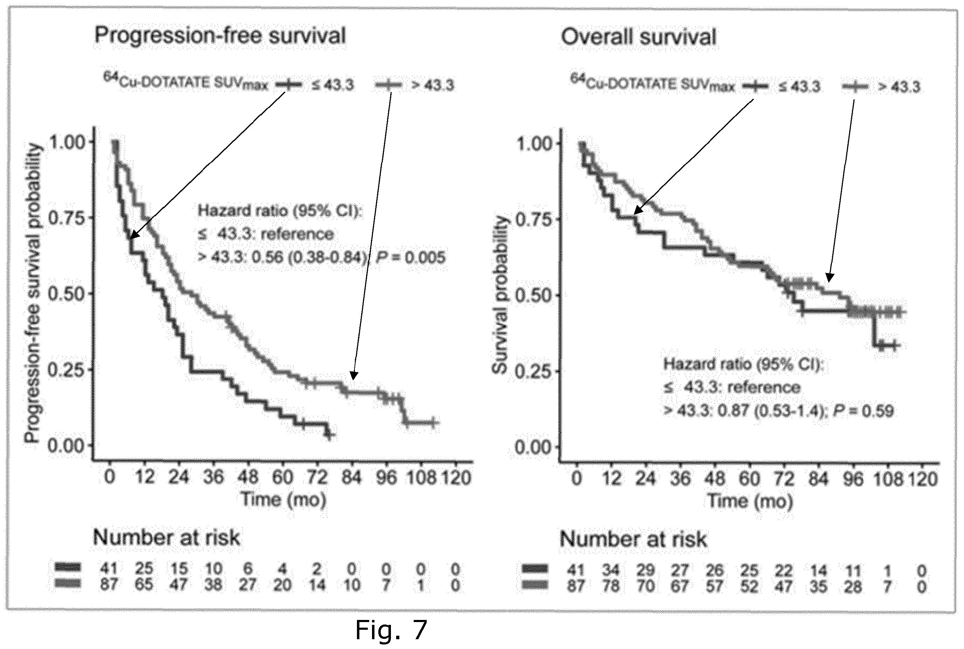

   Figure 7 shows Kaplan-Meier plots of 64Cu-DOTATATE and **(left)** progression free survival (PFS) and **(right)** overall survival (OS) in neuroendocrine tumors patients. From ref. 22 (Carlsen et al. J Nucl Med. 2020). Reference figure, not part of the invention.

The present invention will now be described in more detail in the following.

### Detailed description of the invention

### Definitions

Prior to discussing the present invention in further details, the following terms and conventions will first be defined:

### Neuroendocrine neoplasms (NENs)

"Neuroendocrine neoplasms" (NENs) or "Neuroendocrine tumors" (NETs) originate from the neuroendocrine cells and are found primarily in the gastro-intestinal tract, pancreas and lungs.

Although there are many kinds of NENs, they are largely treated as a group, because the cells of these neoplasms share common features, such as looking similar, having special secretory granules, and often producing biogenic amines and polypeptide hormones.

### Ga68

Gallium-68.

### 64Cu

Copper-64.

### 177Lu

177Lu is a low-energy beta-emitter (~1.5 mm maximum penetration in soft tissue) capable of inducing cytotoxic effects in tumors but not or very limited in surrounding tissue by providing both a 'cross-fire'- and 'bystander' effect from direct betaparticles and Auger electrons, respectively. 177Lu therefore is considered an optimal radionuclide for therapy of large as well as small tumor lesions and/or disseminated metastatic disease.

### AE105

Ac-Asp-Cha-Phe-(D)Ser-(D)Arg-Tyr-Leu-Trp-Ser

The peptide according to the invention can e.g. be synthesized by standard solidphase peptide chemistry.

### NOTA

NOTA: 2,2',2"-(1,4,7-triazacyclononane-1,4,7-triyl)triacetic acid.

NOTA may be coupled to AE105 thereby providing NOTA-AE105 (NOTA-Asp-Cha-Phe-Ser-Arg-Tyr-Leu-Trp-Ser)). This can be illustrated by the following chemical structure:

In an embodiment, the imaging agent is 68Ga-NOTA-AE105.

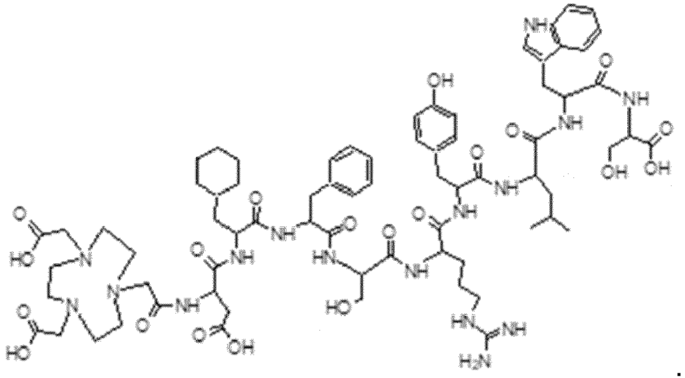

### DOTA

DOTA (also known as tetraxetan) is an organic compound with the formula (CH₂CH₂NCH₂CO₂H)₄. The molecule consists of a central 12-membered tetraaza (i.e., containing four nitrogen atoms) ring. DOTA is used as a complexing agent, especially for lanthanide ions. Its complexes have medical applications as contrast agents and cancer treatments.

### Preferred IUPAC name of DOTA

2,2',2",2"'-(1,4,7,10-Tetraazacyclododecane-1,4,7,10-tetrayl)tetraacetic acid Further below DOTA is shown in complex with 68Ga and 64CU coupled to AE105.

### Relapse-free survival (RFS)

The term "relapse-free survival" (RFS) is defined as clinical endpoint defined as time from diagnosis to any relapse of the disease at the locoregional (TN site) and/or distant metastasis (M site) with deaths from other causes recorded as censoring and disease-free survival (DFS), which is defined as RFS, but includes death of any reason as an event.

### Overall survival (OS)

The term "Overall survival" (OS) is defined as time from diagnosis to death of any cause. Follow-up time was calculated from the time of referral to radiotherapy until first relapse, death or until end of follow-up January 1^{st}, 2021.

### Threshold level

In the context of the present invention, the term "threshold level", "reference level" or "cut-off" relates to a standard in relation to a quantity, which other values or characteristics can be compared to.

In one embodiment of the present invention, it is possible to determine a threshold level by investigating the uPAR levels by PET/CT from healthy subjects. By applying different statistical means, such as cut-off finding, multivariate analysis, one or more threshold level can be calculated.

See also example 5, where cut-offs are determined.

Based on these results, a cut-off may be obtained that shows the relationship between the level(s) detected and patients at risk. The cut-off can thereby be used e.g. to determine the uPAR levels, which for instance corresponds to an increased risk of a poor RFS or OS.

### Risk Assessment

The present inventors have successfully developed a new method to predict the prognosis of a subject with neuroendocrine neoplasms (NENs), such as RFS and/or OS. To determine whether a patient has an increased risk of a poor prognosis, a cut-off (reference level) must be established. This cut-off may be established by the laboratory, the physician or on a case-by-case basis for each patient.

The cut-off level could be established using a number of methods, including: multivariate statistical tests (such as partial least squares discriminant analysis (PLS-DA), random forest, support vector machine, etc.), percentiles, mean plus or minus standard deviation(s); median value; fold changes.

The multivariate discriminant analysis and other risk assessments can be performed on the free or commercially available computer statistical packages (SAS, SPSS, Matlab, R, etc.) or other statistical software packages or screening software known to those skilled in the art.

As obvious to one skilled in the art, in any of the embodiments discussed above, changing the risk cut-off level could change the results of the discriminant analysis for each subject.

Statistics enables evaluation of the significance of each level. Commonly used statistical tests applied to a data set include t-test, f-test or even more advanced tests and methods of comparing data. Using such a test or method enables the determination of whether two or more samples are significantly different or not. The significance may be determined by the standard statistical methodology known by the person skilled in the art.

The chosen reference level may be changed depending on the mammal/subject for which the test is applied.

Preferably, the subject according to the invention is a human subject.

The chosen reference level may be changed if desired to give a different specificity or sensitivity as known in the art. Sensitivity and specificity are widely used statistics to describe and quantify how good and reliable a biomarker or a diagnostic test is. Sensitivity evaluates how good a biomarker or a diagnostic test is at detecting a disease, while specificity estimates how likely an individual (i.e. control, patient without disease) can be correctly identified as not at risk. Several terms are used along with the description of sensitivity and specificity; true positives (TP), true negatives (TN), false negatives (FN) and false positives (FP). If a disease is proven to be present in a sick patient, the result of the diagnostic test is considered to be TP. If a disease is not present in an individual (i.e. control, patient without disease), and the diagnostic test confirms the absence of disease, the test result is TN. If the diagnostic test indicates the presence of disease in an individual with no such disease, the test result is FP. Finally, if the diagnostic test indicates no presence of disease in a patient with disease, the test result is FN.

### Sensitivity

As used herein the sensitivity refers to the measures of the proportion of actual positives, which are correctly identified as such, i.e. the percentage of subjects having a risk of a poor prognosis above normal who are identified as having a poor prognosis above normal.

Usually the sensitivity of a test can be described as the proportion of true positives of the total number with the target disorder i.e. a risk of poor prognosis above normal. All patients with the target disorder are the sum of (detected) true positives (TP) and (undetected) false negatives (FN).

### Specificity

As used herein the specificity refers to measures of the proportion of negatives which are correctly identified - i.e. the percentage of mammal with a non-increased risk of poor prognosis that are identified as not having a risk of poor prognosis above normal. The ideal diagnostic test is a test that has 100 % specificity, i.e. only detects subjects with a risk of having poor prognosis above normal and therefore no false positive results, and 100% sensitivity, i.e. detects all subjects with a risk of having poor prognosis above normal and therefore no false negative results.

For any test, there is usually a trade-off between each measure. For example, in a manufacturing setting in which one is testing for faults, one may be willing to risk discarding functioning components (low specificity), in order to increase the chance of identifying nearly all faulty components (high sensitivity). This trade-off can be represented graphically using a ROC curve.

The chosen specificity determines the percentage of false positive cases that can be accepted in a given study/population and by a given institution. By decreasing specificity an increase in sensitivity is achieved.

As will be generally understood by those skilled in the art, methods for screening for prognosis are processes of decision making and therefore the chosen specificity and sensitivity depends on what is considered to be the optimal outcome by a given institution/clinical personnel.

### SUVmax

In the present context, the term "SUVmax" refers to the "maximum standardized uptake value" (SUVmax), which is widely used for measuring the uptake of uPAR and FDG by malignant tissue. Increased uptake values reflect increased uptake or binding of the tracers to cancer cells, and can be imaged and quantified using PET.

### SUVmean

In the present context the term "SUVmean", refers to the mean standardized uptake value.

### Target-to-liver ratio (TLR)

TLR is defined and calculated as lesion SUVmax /normal liver SUVmean. It was used to define clearly positive lesions, when lesion SUVmax /normal liver SUVmean was greater than 2.

### Companion diagnostic

In the present context, a "companion diagnostic" is a diagnostic test used as a companion to a therapeutic drug to determine its applicability to a specific person.

Companion diagnostics may be co-developed with drugs to aid in selecting or excluding patient groups for treatment with that particular drug based on their biological characteristics that determine responders and non-responders to the therapy. Companion diagnostics are developed based on companion biomarkers, biomarkers that prospectively help predict likely response or severe toxicity.

### Imaging agent for use in the prognostication of a neuroendocrine neoplasm (NEN) patient by PET

Improved prognostication of neuroendocrine neoplasm (NEN) patients are important, e.g. for determining the need for radiotherapy, but also for determining the correct radiotherapy. Thus, improved companion diagnostics are also important. Thus, an aspect of the invention relates to a positron-emitting imaging agent for use in the prognostication of a neuroendocrine neoplasm (NEN) patient by PET imaging of the cancer,
wherein said imaging agent comprises a uPAR binding peptide coupled via the chelating agent NOTA to the radionuclide 68Ga; and
wherein the uPAR binding peptide is (D-Asp)-([beta]-cyclohexyl-L-alanine)-(Phe)-(D-Ser)-(D-Arg)-(Tyr)-(Leu)-(Trp)-(Ser) or a uPAR binding variant thereof.

As outlined in example 5, the imaging agent according to the invention is a strong prognostic marker for NEN patients. Further, optimized cut-offs have been identified. Again, it is noted that such marker is also relevant as a companion diagnostics, since it can be used for identifying patients which are likely susceptible to radiotherapy using a radionuclide coupled to a uPAR binding peptide.

Different uPAR binding variants of the uPAR binding peptide (including AE105) are disclosed in WO2014/086364 A1. Thus, in an embodiment, the uPAR binding variant is selected from the group consisting of
- (D-Asp)-([beta]-cyclohexyl-L-alanine)-(Phe)-(D-Ser)-(D-Arg)-(Tyr)-(Leu)-(Trp)-(Ser),
- (Ser)-(Leu)-([beta]-cyclohexyl-L-alanine)-(Phe)-(D-Ser)-(Gln)-(Tyr)(Leu)-(Trp)-(Ser),
- (D-Glu)-([beta]-cyclohexyl-L-alanine)-(Phe)-(D-Ser)-(D-Tyr)-(Tyr)-(Leu)-(Trp)-(Ser),
- (Asp)-([beta]-cyclohexyl-L-alanine)-(Phe)-(D-Ser)-(D-Arg)-(Tyr)-(Leu)-(Trp)-(Ser),
- (Asp)-([beta]-cyclohexyl-L-alanine)-(Phe)-(Ser)-(D-Arg)-(Tyr)-Leu)-(Trp)-(Ser),
- (D-Asp)-([beta]-cyclohexyl-L-alanine)-(Phe)-(Ser)-(D-Arg)-(Tyr)-Leu)-(Trp)-(Ser),
- (D-Thr)-([beta]-cyclohexyl-L-alanine)-(Phe)-(D-Ser)-(D-Arg)-(Tyr)-(Leu)-(Trp)-(Ser),
- (D-Asp)-([beta]-cyclohexyl-L-alanine)-(Phe)-(D-Ser)-(D-Arg)-(Tyr)-(Leu)-([beta]-2-naphthyl-L-alanine)-(Ser),
- (Asp)-([beta]-cyclohexyl-L-alanine)-(Phe)-(D-Ser)-(Arg)-(Tyr)-(Leu)-(Trp)-(Ser),
- (Asp)-([beta]-cyclohexyl-L-alanine)-(Phe)-(D-Ser)-(D-Arg)-(Tyr)-(Leu)([beta]-1-naphthyl-L-alanine)-(Ser),
- (D-Glu)-([beta]-cyclohexyl-L-alanine)-(Phe)-(D-Ser)-(Tyr)-(Tyr)-(Leu)-(Trp)-(Ser),
- (Asp)-([beta]-cyclohexyl-L-alanine)-(Phe)-(D-Ser)-(D-Arg)-(Leu)-(Leu)-(Trp)-(D-His),
- (Asp)-([beta]-cyclohexyl-L-alanine)-(Phe)-(D-Ser)-(D-Arg)-([beta]-cyclohexyl-L-alanine)-(Leu)-(Trp)-(Ile),
- (Asp)-([beta]-cyclohexyl-L-alanine)-(Phe)-(D-Ser)-(D-Arg)-(Tyr)-(Leu)([beta]-1-naphthyl-L-alanine)-(D-His),
- (Asp)-([beta]-cyclohexyl-L-alanine)-(Phe)-(D-Ser)-(D-Arg)-(N-(2,3-dimethoxybenzyl)glycine)-(D-Phe)-(N-(3-indolylethyl)glycine)-(N-(2-methoxyethyl)glycine),
- (Asp)-([beta]-cyclohexyl-L-alanine)-(Phe)-(D-Ser)-(D-Arg)-(N-(2,3-dimethoxybenzyl)glycine)-(D-Phe)-(N-benzylglycine)-(N-(2[beta]thoxyethyl)glycine),
- (Asp)-([beta]-cyclohexyl-L-alanine)-(Phe)-(D-Ser)-(D-Arg)-(N-(2,3-dimethoxybenzyl)glycine)-(D-Phe)-(N-(methylnaphthalyl)glycine)-(N-(2-methoxyethyl)glycine), and
- (Asp)-([beta]-cyclohexyl-L-alanine)-(Phe)-(D-Ser)-(D-Arg)-(N-(2,3-dimethoxybenzyl)glycine)-(D-Phe)-(N-(2,3-dimethoxybenzyl)glycine)-(Ile).

The uPAR binding part of the imaging agent is preferably the one known as AE105. Thus, in an embodiment, the peptide is (D-Asp)-([beta]-cyclohexyl-L-alanine)-(Phe)-(D-Ser)-(D-Arg)-(Tyr)-(Leu)-(Trp)-(Ser).

In another embodiment, the C-terminal is a carboxylic acid or an amide.

In yet an embodiment, the imaging agent has the formula

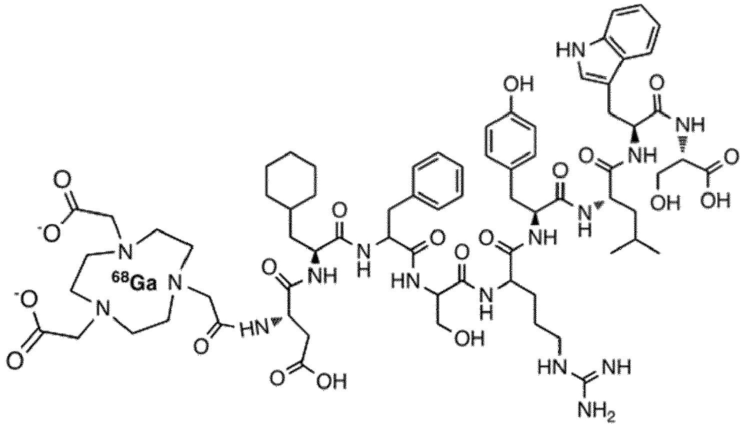

To further improve localization of the cancer, the PET scan may be combined with other scanning types. Thus, in an embodiment, prognostication comprises PET/CT scanning and/or PET/MR scanning. In the example section, PET/CT has been used.

The prognostication may be further defined. Thus, in an embodiment, the prognosis is prognosis of relapse-free survival (RFS) and/or overall survival (OS). In the example section, RFS and OS have been assessed.

Different amount (MBq) of the imaging may be used. Also the time before scanning after administration of the imaging agent may vary. Thus, in an embodiment, the imaging agent is to be administered in a dose of 10-500 MBq followed by PET scanning 10 min to 24 hours after the imaging agent has been administered, and quantification through SUVmax and/or SUVmean and/or TLR.

In another embodiment, the imaging agent is to be administered in a dose of 20-400 MBq, such as 50-400 MBq, such as 70-300 MBq, such as 100-300 MBq or such as 100-300 MBq. preferably such as 150-250 MBq, and more preferably 170-230 MBq. Thus in a preferred embodiment, the imaging agent is to be administered in a dose of 70-300 MBq, preferably such as 150-250 MBq. In the example section, around 200 MBq has been used and PET imaging was performed with a Siemens Biograph 128 mCT. However, other equipment may be more sensitive and therefore allow for lower amount (MBq) of the imaging agent

In an embodiment, a sufficient amount of imaging aging is administered to allow for PET imaging with enough radioactivity dose for imaging.

68Ga has a half-life of around 1 hour (68 min) making 10 minutes to 5 hours, preferably 20 minutes to 3 hours realistic time before PET scanning. 64Cu has a half-life of around 12.7 hours making an interval of 20 minutes to 24 hours before PET scanning realistic.

Thus, in a related embodiment, the PET scanning follows 20 minutes to 10 hours after the imaging agent has been administered, such as 20 minutes to 5 hours, such as 30 minutes to 3 hours after the imaging agent has been administered.

In yet another embodiment, the imaging agent according to the invention is in a pharmaceutical composition comprising the imaging agent, together with one or more pharmaceutical acceptable adjuvants, excipients and/or diluents.

To be able to make a prognostication, a threshold level (cut-off/reference level) may be included. Thus, in an embodiment, the imaging agent for use according to the invention,
∘ a SUVmax and/or SUVmean and/or target-to liver ratio (TLR) quantification level above a threshold level is indicative of a poor Relapse-free survival (RFS) prognosis and/or poor overall survival (OS) prognosis; and
∘ a SUVmax and/or SUVmean and/or target-to liver ratio (TLR) quantification level equal to or below a threshold level is indicative of good Relapse-free survival (RFS) prognosis and/or good overall survival (OS) prognosis.

In example 5, specific optimal threshold levels (cut-offs) have been calculated.

In yet an embodiment, said target-to liver ratio (TLR) threshold level is in the range 1.1-4, such as in the range 1.3-4, such as in the range 2-3. uPAR TLR dichotomized at median was significantly associated with PFS and OS where patients with a high uPAR expression had a significantly worse prognosis (Example 5, Table 4 and 5).

In yet another embodiment, said target-to liver ratio (TLR) threshold level is in the range 1.1-2, such as in the range 1.1-1.5, such as in the range 1.2-1.4. By using a lower cutoff of TLR (1.32) a smaller group of patients (n=10) with no or a very low risk of death or progression could be identified (Example 5, Figure 4).

In another embodiment, said SUVmax and/or SUVmean threshold level is in the range 1-4, such as in the range 2-4, preferably in the range 2-3, such as 2-2.5. As outlined in example 5 (figure 5A-B), SUV threshold levels could also be established albeit not being superior to TLR thresholds.

In yet an embodiment, said threshold level is determined by using a cut-off finding method to obtain a split in a Kaplan-Meier plot (log-rank test) and the corresponding hazard ratios (HRs).

In an embodiment, the neuroendocrine neoplasm (NEN) is classified as NET G1, NET G2, NET G3 or NEC according to WHO classification (1). Example 3 shows that uPAR PET can identify low grade (NET G1/G2) and high grade NEN (NET G3 and NEC).

It is of course noted that embodiments of other aspects of the invention is also applicable to this aspect.

### A method of in vivo imaging by PET imaging, for assessing the prognosis of a Neuroendocrine neoplasm (NEN)

Another aspect of the invention relates to a method of in vivo imaging by PET imaging, for assessing the prognosis of a Neuroendocrine neoplasm (NEN) in a patient, said method comprising:
a) provision of a subject to which a imaging agent according to the invention has been previously administered;
b) detecting by in vivo PET imaging the radioactive emissions from the radioisotope of the administered imaging agent of step a);
c) generating an image representative of the location and/or amount of said radioactive emissions;
d) determining the distribution and extent of uPAR expression in said subject, wherein said expression is correlated with said signals emitted by said in vivo imaging agent; and
e) comparing the determined distribution and extent of uPAR expression to a threshold level;
   ▪ wherein a SUVmax and/or SUVmean and/or TLR quantification level above a threshold level is indicative of a poor Relapse-free survival (RFS) prognosis and/or poor overall survival (OS) prognosis; and
   ▪ wherein a SUVmax and/or SUVmean and/or TLR quantification level equal to or below a threshold level is indicative of good Relapse-free survival (RFS) prognosis and/or good overall survival (OS) prognosis.

In an embodiment, if a good Relapse-free survival (RFS) prognosis or good overall survival (OS) prognosis is indicated, said subject will be scheduled for a de-escalated treatment regimen to avoid unnecessary toxicity and/or for a risk stratified follow-up schedule. In the present context, a de-escalated treatment protocol is a protocol, which is considered less toxic/harmful to the patient compared to a patient who has received a poor Relapse-free survival (RFS) prognosis.

In an embodiment, said de-escalated treatment being abstaining from or reducing radiotherapy.

In another embodiment, if a poor Relapse-free survival (RFS) prognosis or poor overall survival (OS) prognosis is indicated, said subject is scheduled for radiotherapy.

In yet an embodiment, wherein said radiotherapy is with a radiopharmaceutical comprises a radionuclide and a uPAR binding peptide, such as linked via a chelator.

In yet another embodiment, said treatment is any anticancer therapy.

In a further embodiment, a level expressed as SUVmax, SUVmean and/or TLR determines if a patient is eligible for targeted radionuclide therapy.

In an aspect, the method forms the basis as a companion diagnostics for targeted radionuclide therapy. Thus, in such as an aspect
∘ a SUVmax and/or SUVmean and/or target-to liver ratio (TLR) quantification level above a threshold level is indicative of that a uPAR binding drug, such as a uPAR binding radiopharmaceutical will be effective against said NEN; and
∘ a SUVmax and/or SUVmean and/or target-to liver ratio (TLR) quantification level equal to or below a threshold level is indicative of that a uPAR binding drug, such as a uPAR binding radiopharmaceutical will not be effective against said NEN.

Preferably, the drug is a uPAR binding radiopharmaceutical, more preferably the radiopharmaceutical is 177Lu-DOTA-AE105.

### Use of a positron-emitting imaging agent in the prognosis of a neuroendocrine neoplasm (NEN) in a patient by in vivo PET imaging

A further aspect of the invention relates to the use of a positron-emitting imaging agent according to the invention in the prognosis of neuroendocrine neoplasm (NEN) patients by in vivo PET imaging of uPAR expressing tumors,
wherein said imaging agent comprises a uPAR binding peptide coupled via the chelating agent NOTA to the radionuclide 68Ga;
wherein the uPAR binding peptide is (D-Asp)-([beta]-cyclohexyl-L-alanine)-(Phe)-(D-Ser)-(D-Arg)-(Tyr)-(Leu)-(Trp)-(Ser) or a uPAR binding variant thereof.

In an alternative part of this aspect, said imaging agent comprises a uPAR binding peptide coupled via the chelating agent NOTA or DOTA to the radionuclide 68Ga or 64Cu.

Again, in an embodiment, the uPAR binding variant thereof is selected from the group consisting of
- (D-Asp)-([beta]-cyclohexyl-L-alanine)-(Phe)-(D-Ser)-(D-Arg)-(Tyr)-(Leu)-(Trp)-(Ser),
- (Ser)-(Leu)-([beta]-cyclohexyl-L-alanine)-(Phe)-(D-Ser)-(Gln)-(Tyr)(Leu)-(Trp)-(Ser),
- (D-Glu)-([beta]-cyclohexyl-L-alanine)-(Phe)-(D-Ser)-(D-Tyr)-(Tyr)-(Leu)-(Trp)-(Ser),
- (Asp)-([beta]-cyclohexyl-L-alanine)-(Phe)-(D-Ser)-(D-Arg)-(Tyr)-(Leu)-(Trp)-(Ser),
- (Asp)-([beta]-cyclohexyl-L-alanine)-(Phe)-(Ser)-(D-Arg)-(Tyr)-Leu)-(Trp)-(Ser),
- (D-Asp)-([beta]-cyclohexyl-L-alanine)-(Phe)-(Ser)-(D-Arg)-(Tyr)-Leu)-(Trp)-(Ser),
- (D-Thr)-([beta]-cyclohexyl-L-alanine)-(Phe)-(D-Ser)-(D-Arg)-(Tyr)-(Leu)-(Trp)-(Ser),
- (D-Asp)-([beta]-cyclohexyl-L-alanine)-(Phe)-(D-Ser)-(D-Arg)-(Tyr)-(Leu)-([beta]-2-naphthyl-L-alanine)-(Ser),
- (Asp)-([beta]-cyclohexyl-L-alanine)-(Phe)-(D-Ser)-(Arg)-(Tyr)-(Leu)-(Trp)-(Ser),
- (Asp)-([beta]-cyclohexyl-L-alanine)-(Phe)-(D-Ser)-(D-Arg)-(Tyr)-(Leu)([beta]-1-naphthyl-L-alanine)-(Ser),
- (D-Glu)-([beta]-cyclohexyl-L-alanine)-(Phe)-(D-Ser)-(Tyr)-(Tyr)-(Leu)-(Trp)-(Ser),
- (Asp)-([beta]-cyclohexyl-L-alanine)-(Phe)-(D-Ser)-(D-Arg)-(Leu)-(Leu)-(Trp)-(D-His),
- (Asp)-([beta]-cyclohexyl-L-alanine)-(Phe)-(D-Ser)-(D-Arg)-([beta]-cyclohexyl-L-alanine)-(Leu)-(Trp)-(Ile),
- (Asp)-([beta]-cyclohexyl-L-alanine)-(Phe)-(D-Ser)-(D-Arg)-(Tyr)-(Leu)([beta]-1-naphthyl-L-alanine)-(D-His),
- (Asp)-([beta]-cyclohexyl-L-alanine)-(Phe)-(D-Ser)-(D-Arg)-(N-(2,3-dimethoxybenzyl)glycine)-(D-Phe)-(N-(3-indolylethyl)glycine)-(N-(2-methoxyethyl)glycine),
- (Asp)-([beta]-cyclohexyl-L-alanine)-(Phe)-(D-Ser)-(D-Arg)-(N-(2,3-dimethoxybenzyl)glycine)-(D-Phe)-(N-benzylglycine)-(N-(2[beta]thoxyethyl)glycine),
- (Asp)-([beta]-cyclohexyl-L-alanine)-(Phe)-(D-Ser)-(D-Arg)-(N-(2,3-dimethoxybenzyl)glycine)-(D-Phe)-(N-(methylnaphthalyl)glycine)-(N-(2-methoxyethyl)glycine), and
- (Asp)-([beta]-cyclohexyl-L-alanine)-(Phe)-(D-Ser)-(D-Arg)-(N-(2,3-dimethoxybenzyl)glycine)-(D-Phe)-(N-(2,3-dimethoxybenzyl)glycine)-(Ile).

### Companion diagnostic

As outlined above, the methods of the invention and the compositions for use according to the invention, may also find use as a companion diagnostic. Thus, a further aspect of the invention relates to a positron-emitting imaging agent for use as companion diagnostic of a neuroendocrine neoplasm (NEN) patient by PET imaging of the cancer,
wherein said imaging agent comprises a uPAR binding peptide coupled via the chelating agent NOTA or DOTA to the radionuclide 68Ga or 64Cu; and
wherein the uPAR binding peptide is (D-Asp)-([beta]-cyclohexyl-L-alanine)-(Phe)-(D-Ser)-(D-Arg)-(Tyr)-(Leu)-(Trp)-(Ser) or a uPAR binding variant thereof;
wherein
   ∘ a SUVmax and/or SUVmean and/or target-to liver ratio (TLR) quantification level above a threshold level is indicative of that a uPAR binding drug, such as a uPAR binding radiopharmaceutical will be effective against said NEN; and
   ∘ a SUVmax and/or SUVmean and/or target-to liver ratio (TLR) quantification level equal to or below a threshold level is indicative of that a uPAR binding drug, such as a uPAR binding radiopharmaceutical will not be effective against said NEN.

In a preferred embodiment, the imaging agent is 68Ga-NOTA-AE105. This imaging agent has been used in the example section.

In another preferred embodiment, the drug is a radiopharmaceutical. In an even more preferred embodiment, the uPAR binding radiopharmaceutical is 177Lu-DOTA-AE105.

Again, it is noted that embodiments of other aspects of the invention is also applicable to this aspect.

### Radiopharmaceutical for use in the treatment or alleviation of a neuroendocrine neoplasm (NEN) in a subject

As outlined above, the imaging agent may be used as a companion diagnostic. In this way subtypes of NEN can be identified which expresses certain upregulated or downregulated surface proteins. Upregulated surface proteins may be used to target radiopharmaceutical to a cancer.

Thus, yet an aspect of the invention relates to a composition comprising a radiopharmaceutical for use in the treatment or alleviation of a neuroendocrine neoplasm (NEN) in a subject
wherein said radiopharmaceutical comprises a radionuclide and a uPAR binding peptide; and
wherein the uPAR binding peptide is (D-Asp)-([beta]-cyclohexyl-L-alanine)-(Phe)-(D-Ser)-(D-Arg)-(Tyr)-(Leu)-(Trp)-(Ser) or a uPAR binding variant thereof; and
wherein said subject has been diagnosed with a uPAR expressing neuroendocrine neoplasm (NEN); and wherein said uPAR expression is above a predetermined threshold level,
wherein said threshold level is determined using a positron-emitting imaging agent according to the invention.

Preferably, the radionuclide and the peptide are linked via a chelator, such as DOTA or NOTA.

In an embodiment, the radionuclide is for targeted radionuclide therapy (alpha, beta-emitters or auger) and is selected from the group of isotopes consisting of 67Cu, 177Lu, 89Sr, 90Y, 117mSn, 131I, 153Sm, 166Ho, 186Re, 188Re, 211At, 212Pb, 212Bi, 213Bi, 223Ra, 224Ra, 225Ac, 227Th, preferably selected from 177Lu, 67Cu, 90Y, 211At, 225Ac, and 227Th, and more preferably being 177Lu.

In another embodiment, said radiopharmaceutical is coupled to the uPAR binding peptide via a chelator, such as the chelator being selected from the group consisting of DOTA, CB-DO2A, 3p-C-DEPA, TCMC, Oxo-DO3A, TETA, TE2A, CB-TE2A, CB-TE1A1P, CB-TE2P, MM-TE2A, DM-TE2A, SarAr, SarAr-NCS, diamSar, AmBaSar, BaBaSar, ATSM, CB-TE1A1P and CB-TE2P, NOTA, NETA, TACN-TM, NODAGA, TRAP, AAZTA , DATA, H2dedpa, CP256, PCTA, THP, DTPA, 1B4M-DTPA, CHX-A"-DTPA, TRAP (PRP9), NOPO, DFO HOPO, H6phospa, PCTA, H2dedpa, H4octapa, H2azapa, H5decapa, HBED, HBED-cc, SHBED, BPCA, CP256, HEHA, PEPA and RESCA1, preferably from any of DOTA, NOTA, CB-TE2A, NODAGA, DFO, HBED, and HBED-cc. More, preferably the chelator is DOTA or NOTA.

In an embodiment, said target-to liver ratio (TLR) threshold level is in the range 1.1-4, such as in the range 1.3-4, such as in the range 2-4 or 2-3,

In another embodiment, said target-to liver ratio (TLR) threshold level is in the range 1.1-2, such as in the range 1.1-1.5, such as in the range 1.2-1.4.

In yet an embodiment, radiopharmaceutical is 177Lu-DOTA-AE105.

In yet another embodiment, the radiopharmaceutical has the formula:

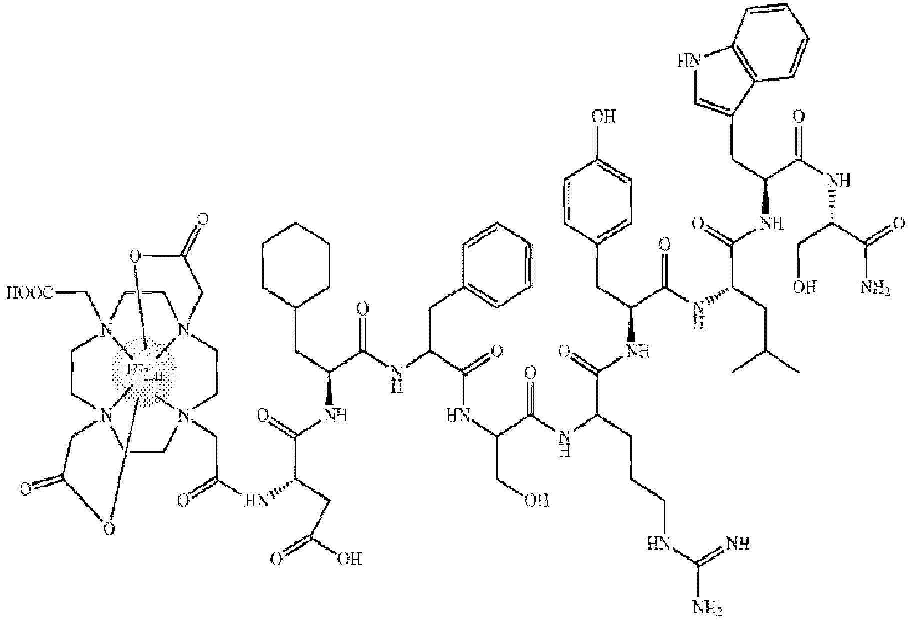

Again, it is noted that embodiments of other aspects of the invention is also applicable to this aspect.

### Additional aspects of the invention

An additional aspect of the invention relates to a positron-emitting imaging agent for use in the prognostication of a neuroendocrine neoplasm (NEN) patient by PET imaging of the cancer,
wherein said imaging agent comprises a uPAR binding peptide coupled via the chelating agent NOTA or DOTA to the radionuclide 68Ga or 64Cu; and
wherein the uPAR binding peptide is (D-Asp)-([beta]-cyclohexyl-L-alanine)-(Phe)-(D-Ser)-(D-Arg)-(Tyr)-(Leu)-(Trp)-(Ser) or a uPAR binding variant thereof.

Again, in an embodiment, uPAR binding variant thereof is selected from the group consisting of
- (D-Asp)-([beta]-cyclohexyl-L-alanine)-(Phe)-(D-Ser)-(D-Arg)-(Tyr)-(Leu)-(Trp)-(Ser),
- (Ser)-(Leu)-([beta]-cyclohexyl-L-alanine)-(Phe)-(D-Ser)-(Gln)-(Tyr)(Leu)-(Trp)-(Ser),
- (D-Glu)-([beta]-cyclohexyl-L-alanine)-(Phe)-(D-Ser)-(D-Tyr)-(Tyr)-(Leu)-(Trp)-(Ser),
- (Asp)-([beta]-cyclohexyl-L-alanine)-(Phe)-(D-Ser)-(D-Arg)-(Tyr)-(Leu)-(Trp)-(Ser),
- (Asp)-([beta]-cyclohexyl-L-alanine)-(Phe)-(Ser)-(D-Arg)-(Tyr)-Leu)-(Trp)-(Ser),
- (D-Asp)-([beta]-cyclohexyl-L-alanine)-(Phe)-(Ser)-(D-Arg)-(Tyr)-Leu)-(Trp)-(Ser),
- (D-Thr)-([beta]-cyclohexyl-L-alanine)-(Phe)-(D-Ser)-(D-Arg)-(Tyr)-(Leu)-(Trp)-(Ser),
- (D-Asp)-([beta]-cyclohexyl-L-alanine)-(Phe)-(D-Ser)-(D-Arg)-(Tyr)-(Leu)-([beta]-2-naphthyl-L-alanine)-(Ser),
- (Asp)-([beta]-cyclohexyl-L-alanine)-(Phe)-(D-Ser)-(Arg)-(Tyr)-(Leu)-(Trp)-(Ser),
- (Asp)-([beta]-cyclohexyl-L-alanine)-(Phe)-(D-Ser)-(D-Arg)-(Tyr)-(Leu)([beta]-1-naphthyl-L-alanine)-(Ser),
- (D-Glu)-([beta]-cyclohexyl-L-alanine)-(Phe)-(D-Ser)-(Tyr)-(Tyr)-(Leu)-(Trp)-(Ser),
- (Asp)-([beta]-cyclohexyl-L-alanine)-(Phe)-(D-Ser)-(D-Arg)-(Leu)-(Leu)-(Trp)-(D-His),
- (Asp)-([beta]-cyclohexyl-L-alanine)-(Phe)-(D-Ser)-(D-Arg)-([beta]-cyclohexyl-L-alanine)-(Leu)-(Trp)-(Ile),
- (Asp)-([beta]-cyclohexyl-L-alanine)-(Phe)-(D-Ser)-(D-Arg)-(Tyr)-(Leu)([beta]-1-naphthyl-L-alanine)-(D-His),
- (Asp)-([beta]-cyclohexyl-L-alanine)-(Phe)-(D-Ser)-(D-Arg)-(N-(2,3-dimethoxybenzyl)glycine)-(D-Phe)-(N-(3-indolylethyl)glycine)-(N-(2-methoxyethyl)glycine),
- (Asp)-([beta]-cyclohexyl-L-alanine)-(Phe)-(D-Ser)-(D-Arg)-(N-(2,3-dimethoxybenzyl)glycine)-(D-Phe)-(N-benzylglycine)-(N-(2[beta]thoxyethyl)glycine),
- (Asp)-([beta]-cyclohexyl-L-alanine)-(Phe)-(D-Ser)-(D-Arg)-(N-(2,3-dimethoxybenzyl)glycine)-(D-Phe)-(N-(methylnaphthalyl)glycine)-(N-(2-methoxyethyl)glycine), and
- (Asp)-([beta]-cyclohexyl-L-alanine)-(Phe)-(D-Ser)-(D-Arg)-(N-(2,3-dimethoxybenzyl)glycine)-(D-Phe)-(N-(2,3-dimethoxybenzyl)glycine)-(Ile).

In an embodiment, the imaging agent comprises 68Ga-NOTA and the uPAR binding peptide as outlined for the invention.

In an embodiment, the imaging agent comprises 68Ga-DOTA and the uPAR binding peptide as outlined for the invention. In an embodiment, the imaging agent has the formula:

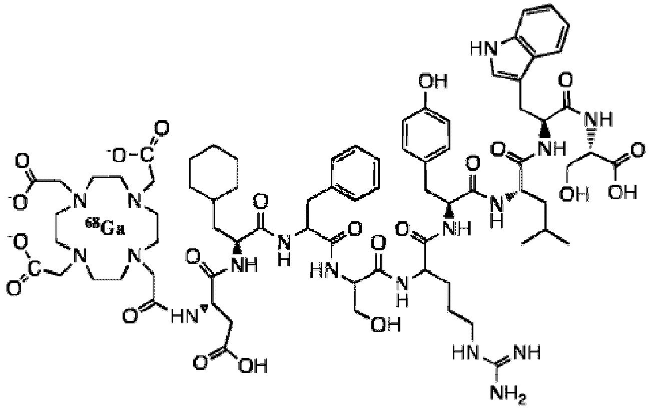

In an embodiment, the imaging agent comprises 64Cu-NOTA and the uPAR binding peptide as outlined for the invention. In an embodiment, the imaging agent has the formula:

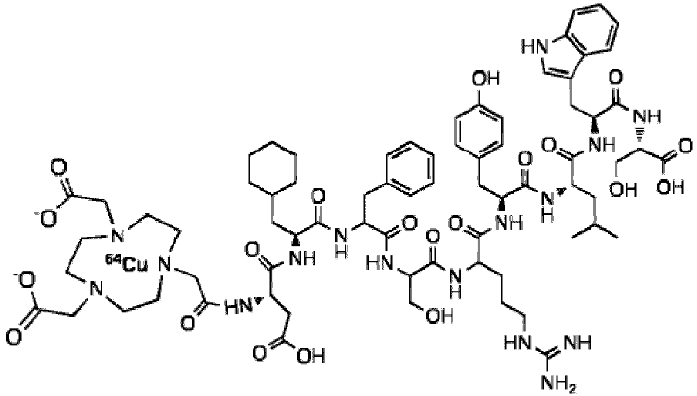

In an embodiment, the imaging agent comprises 64Cu-DOTA and the uPAR binding peptide as outlined for the invention. In an embodiment, the imaging agent has the formula:

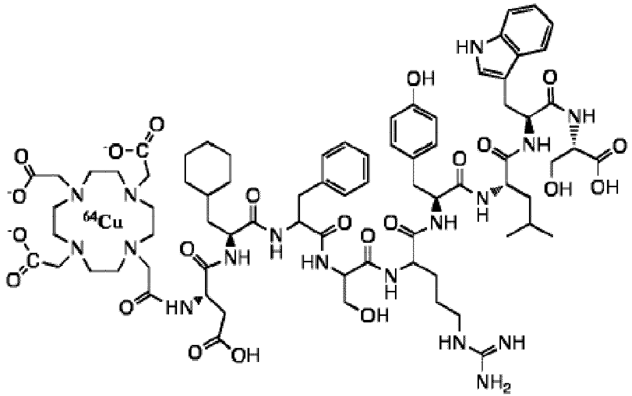

The different variants of the radionuclide-chelator-uPAR binding peptide (including AE105) are also disclosed in WO2014/086364 A1, which is incorporated in here for reference. The two uPAR-PET tracers 64Cu-DOTA-AE105 and ⁶⁸Ga-NOTA-AE105 share the same binding moiety, the peptide AE105. In addition, they have both been tested in both humans and animals (see reference (7), (28) and WO2014/086364 A1) and have demonstrated similar uptake in breast, bladder and prostate cancer. Accordingly, without being bound by theory, it is believed the two PET tracers will work in a similar manner also for NEN patients.

It should be noted that embodiments and features described in the context of one of the aspects of the present invention also apply to the other aspects of the invention.

All patent and non-patent references cited in the present application, are hereby incorporated by reference in their entirety.

The invention will now be described in further details in the following non-limiting examples.

### Examples

### Example 1 - Materials and methods

### Patients

Patients with histologically confirmed NEN were included from the Dept. of Endocrinology (managing low grade NEN, Ki67 ≤20%) and Dept. of Oncology (managing high grade NEN, Ki67 >20%), Copenhagen University Hospital - Rigshospitalet, Denmark between November 17th 2017 - June 29th 2020. Rigshospitalet is a certified Neuroendocrine Tumor Center of Excellence by the European Neuroendocrine Tumor Society. The study was conducted in accordance with the Helsinki Declaration and Good Clinical Practice.

Eligible patients were aged above 18 years and should be able to read and understand the patient information in Danish and to give informed consent, diagnosed with gastro-entero-pancreatic NEN of all grades or broncho-pulmonary NEN, and have a WHO performance status of 0-2. Patients were excluded if pregnant/breast-feeding, had a body mass > 140 kg, a history of allergic reaction attributable to compounds of similar chemical or biologic composition to ⁶⁸Ga-NOTA-AE105 or in case of broncho-pulmonary NEN if the subtype was small cell lung cancer. After signature of written informed consent was obtained, the patients were referred for ⁶⁸Ga-NOTA-AE105 PET/CT at first given opportunity.

### Image acquisition

Data acquisition was performed using a Biograph 128 mCT PET/CT (Siemens Medical Solutions) with an axial field of view of 216 mm. Based on the previous phase I trial (7), the scan was acquired 20 minutes after intravenous administration of approximately 200 MBq ⁶⁸Ga-NOTA-AE105.Tracer production was performed as previously described (7). Whole-body PET scans (mid of orbita to mid of thigh) were acquired with an acquisition time of 4 min per bed position. Attenuation- and scatter- corrected PET data were reconstructed iteratively using a 3D ordinary Poisson ordered-subset expectation-maximization algorithm including point-spread function and time-of-flight information using the TrueX algorithm (Siemens Medical Solutions); the settings were 2 iterations, 21 subsets, 2-mm Gaussian filter. A diagnostic CT scan was obtained before PET scan, with a 2-mm slice thickness, 120 kV, and a quality reference of 225 mAs modulated by the Care Dose 4D automatic exposure control system (Siemens Medical Solutions). An automatic injection system was used to administer 75 mL of an iodine containing contrast agent (Optiray 300; Covidien) for arterial and venous phase CT.

### Image analysis

An experienced board certified nuclear medicine physician together with an experienced board certified radiologist analyzed side-by-side the PET/CT scans. The readers were blinded to patient data. Lesions were identified on CT and/or PET. SUV was calculated as decay-corrected measured radioactivity concentration/(injected activity/body weight). If more than one lesion was present in an organ, the lesion with the highest ⁶⁸Ga-NOTA-AE105 SUVₘₐₓ was noted. If no uPAR positive lesions were identified, but lesions were visible on CT, the largest lesion (based on viable tumor) on CT was used as guide for delineation on the PET scan and SUVₘₐₓ was determined. Physiological liver uptake was assessed in all patients' normal liver tissue, preferable in the right side of the liver avoiding major blood vessels. To standardize measurement of uPAR expression within and between patients, uPAR target-to-liver ratio (uPAR TLR) was used to define a lesion as uPAR positive when lesion SUVₘₐₓ /normal liver SUVₘₑₐₙ ≥ 2.

### Follow-up

The patients were followed at the Rigshospitalet Neuroendocrine Tumor Center of Excellence with regularly visits including clinical examination, blood samples and imaging (CT, MR, ultrasound and/or PET/CT). The frequency was in accordance with ENETS guidelines (13). Follow-up for endpoints was performed on July 8th 2021. Routine CT and or magnetic resonance imaging were used for evaluation of PFS in accordance with Response Evaluation Criteria in Solid Tumors v. 1.1 *(14).* PFS was calculated as time from uPAR PET/CT to, if any, progression or death from any cause. If no progression or death from any cause occurred within the follow-up, the patient was censored at the time of last available diagnostic imaging. OS was calculated as time from uPAR PET/CT to death by any cause. As all death but two were directly related to NEN, we refrained from analyzing disease specific survival. Patients alive at follow-up were censored to the day of follow-up, i.e. July 8th 2021.

### Statistics

Calculation of sample size was based on previous studies of prognostic markers in patients with NEN (15,16), where a 1-year follow-up of 100 patients was sufficient to detect significant differences in PFS and OS among groups (with a risk of Type I error of 0.05 and power of 0.8). To account for dropouts, 120 patients were included. Continuous variables are reported as mean ± standard deviation (SD) or median with range. Kaplan Meier analyses were used for estimation of time to outcome (PFS and OS) and using reverse Kaplan Meier analysis to estimate median follow-up time. We used the Cutoff Finder application to determine the optimal cutoff for uPAR TLR (17). Univariate and multivariate Cox regression analyses for OS and PFS with predictor variables being uPAR TLR and WHO grade were performed. A p-value<0.05 was considered statistically significant. R version 3.6.0 (R Foundation for Statistical Computing, Vienna, Austria) was used for the analyses.

### Example 2 - Patients and image acquisition

We included 120 consecutive patients, whereof 21 patients did not undergo uPAR PET/CT (worsening of disease, n =5; withdrew consent, n=5; did not fulfill inclusion criteria, n= 2; died before uPAR PET/CT, n = 4; not possible to perform uPAR PET/CT due to COVID-19 restrictions, n= 5). Of 99 patients scanned with uPAR PET/CT, 96 patients had evaluable lesions (uPAR PET/CT failed due to technical issue, n = 1; no lesions visible on either CT or PET, n=2). Patients demographic data for study cohort (n=96) are given in Table 1. The majority of patients had small intestinal NEN (64%, 61/96) and 90% (86/96) had metastatic disease. Also, patients with high grade disease were well represented in the cohort with 9% NET G3 (9/96) and 16% NEC (15/96).

**Table 1: Baseline characteristics of patients with neuroendocrine neoplasms.**

| **Base line characteristics** | | **(N=96)** |
|---|---|---|
| Median age (y) | | 66 (range, 34- |

| Gend er | | |
|---|---|---|
| | Female | 39 (41%) |
| | Male | 57 (59%) |

| Site of primary tumor | | |
|---|---|---|
| | Small intestine | 61 (64%) |
| | Pancreas | 15 (16%) |
| | Colon | 10 (10%) |
| | Lung | 6 (6%) |
| | Esophagus | 1 (1%) |
| | Stomach | 2 (2%) |
| | Rectum | 1 (1%) |
| Meta static disease | | 86 (90%) |
| Liver metastases | | 73 (76%) |
| Median Ki67 | | 7 (range, 1-100) |

| World Health Organization grade | | |
|---|---|---|
| | NET G1 | 21 (22%) |
| | NET G2 | 51 (53%) |
| | NET G3 | 9 (9%) |
| | NEC | 15 (16%) |
| Median time from diagnose to uPAR PET/CT (mo) | | 25 (range, 0.5- |
| Prim ary tumor resected | | 37 (39%) |

| Ongo ing treatment at uPAR PET/CT scan time^{∗} | | |
|---|---|---|
| | Somatostatin analogue | 70 (73%) |
| | Interferon | 8 (8%) |
| | Carboplatin or etoposide | 19 (20%) |
| | Capecitabine/5FU | 6 (6%) |
| | Streptozotocin | 5 (5%) |
| | Temozolomide | 2 (2%) |
| | Everolimus | 2 (2%) |

| Com pleted treatment before uPAR PET/CT^{∗} | | |
|---|---|---|
| | On 1. line of therapy | 45 (47%) |
| | Peptide receptor radionuclide therapy | 28 (29%) |
| | Temozolomide | 6 (6%) |
| | Capecitabine/5FU | 6 (6%) |
| | Streptozotocin | 5 (5%) |
| | Carboplatin or etoposide | 10 (10%) |
| | Everolimus or sunitinib | 2 (2%) |
| | Interferon | 6 (6%) |
| | Liver radiofrequency ablation or | 4 (4%) |
| | Resection of liver metastases | 4 (4%) |

| | | |
|---|---|---|
| ^{∗}Some patients had received more than one treatment, therefore the number of treatments exceed the number of patients. Data are number followed by percentage in parentheses, unless otherwise indicated. Percentages were rounded and may not add up to 100%. NET: neuroendocrine tumor. NEC: neuroendocrine carcinoma. | | |

Patients were injected with a median (range) of 194 (104-236) MBq ⁶⁸Ga-NOTA-AE105 and 17.2 (8.7-39.8) µg AE105. Time from injection to PET scan was a median (range) of 22 (18-38) minutes. One patient experienced an adverse event (mild nausea), which was deemed unrelated to ⁶⁸Ga-NOTA-AE105 injection. No serious adverse events were recorded.

### Example 3 - Image analysis

The tracer uptake in normal liver tissue was used as reference for tumor uptake. The mean ± SD normal liver SUVₘₑₐₙ was 1.50±0.39. uPAR positive lesions were seen in both patients with low grade (NET G1/G2) and high grade NEN (NET G3 and NEC) (Table 2). Representative examples of uPAR PET are shown in Figure 1.

**Table 2: Proportion of patients with uPAR PET positive tumors by WHO grades.**

| | G1 (n=21) | G2 (n=51) | G3 (n=24) | Overall (n=96) |
|---|---|---|---|---|
| uPAR PET positive | 12 (57%) | 35 (69%) | 18 (75%) | 65 (68%) |

Data are number followed by percentage in parentheses. uPAR target-to-liver ratio was used to define lesions as uPAR positive when lesion SUVₘₐₓ /normal liver SUVₘₑₐₙ ≥ 2. Of patients with G3 were 8 of 9 NET G3 and 10 of 15 NEC uPAR PET positive.

### Conclusion

Thus, uPAR PET can identify low grade (NET G1/G2) and high grade NEN (NET G3 and NEC).

### Example 4 - Follow-up

Median follow-up time after uPAR PET/CT was 28 months. During follow-up, 59 (62%) patients experienced progressive disease and median PFS was 17.3 months and 28 patients (30%) died. The patient's treatments after uPAR PET/CT are given in Table 3. Treatment with somatostatin analogue was the most frequent (77%, 74/96), and 28% (27/96) of all patients underwent peptide receptor radionuclide therapy during the follow-up period.

**TABLE 3: Treatment after uPAR PET/CT.**

| **Treatment after uPAR/PET (N=96)** | | | |
|---|---|---|---|
| Somatostatin analog | 74 (77%) | Telotristat | 2 (2%) |
| Interferon | 6 (6%) | Topotecan | 2 (2%) |
| Peptide receptor radionuclide therapy | 27 (28%) | Docetaxel | 2 (2%) |
| Capecitabine/5FU | 10 (10%) | Irinotecan | 1 (1%) |
| Everolimus or sunitinib | 11 (11%) | Surgery | 9 (9%) |
| Temozolomide | 8 (8%) | Liver embolization | 7 (7%) |
| Carboplatin or etoposide | 11 (11%) | Liver radiofrequency ablation | 2 (2%) |
| Streptozotocin | 3 (3%) | I External radiation | 11 (11%) |

Some patients had received more than one treatment, therefore the number of treatments exceed the number of patients. Data are number followed by percentage in parentheses.

### Example 5 - Progression-free survival and overall survival

### Aim of study

To determine Progression-free survival (PFS) and overall survival (OS), based on uPAR imaging.

### Results

uPAR TLR as a continuous variable was significantly associated with PFS with a HR (95% CI) of 1.27 (1.02-1.60), p=0.04.

For OS, uPAR TLR as a continuous marker was borderline significant with a HR (95% CI) of 1.37 (0.98-1.92), p=0.06). TLR was then dichotomized at the median value (2.47) for Kaplan Meier analyses (Figure 2). Median OS was not reached in the group with low uPAR expression (TLR < median), and was 32.1 months (23.8; upper limit not reached) in the group with high uPAR expression (TLR ≥ median). Median PFS was 22.1 months (14.7; upper limit not reached) for patients with low uPAR expression and 14.1 months (11.4-22.4) patients with high uPAR expression. uPAR TLR dichotomized at median was significantly associated with PFS and OS where patients with a high uPAR expression had a significantly worse prognosis (Table 4 and 5).

**TABLE 4: Uni- and multivariate Cox regression analyses for progression-free survival.**

| **Prog ression-free survival** | | **Univariate Cox** | | **Multivariate Cox** | |
|---|---|---|---|---|---|
| | | **HR (95% CI)** | **P-value** | **HR (95% CI)** | **P-value** |

| uPAR TLR^{∗} | | | | | |
|---|---|---|---|---|---|
| | Low | *Reference* | - | *Reference* | - |
| | High | 1.87 (1.11-3.17) | 0.02 | 1.75 (1.02-2.99) | 0.04 |

| WHO grades | | | | | |
|---|---|---|---|---|---|
| | NET G1 | *Reference* | | *Reference* | |
| | NET G2 | 1.21 (0.57-2.55) | 0.62 | 1.26 (0.59-2.66) | 0.55 |
| | NET G3 | 4.16 (1.52-11.36) | <0.01 | 3.56 (1.29-9.82) | 0.01 |
| | NEC | 4.26 (1.82-9.95) | <0.001 | 4.43 (1.89-10.39) | <0.001 |

| | | | | | |
|---|---|---|---|---|---|
| ^{∗}uPAR TLR is dichotomized at median (2.47). TLR: target-to-liver ratio. NET: neuroendocrine tumor. NEC: neuroendocrine carcinoma. HR: hazard ratio. CI: confidence interval | | | | | |

**TABLE 5: Uni- and multivariate Cox regression analyses for overall survival.**

| **Overall survival** | | **Univariate Cox** | | **Multivariate Cox** | |
|---|---|---|---|---|---|
| | | **HR (95% CI)** | **P-value** | **HR (95% CI)** | **P-value** |
| uPAR TLR^{∗} | | | | | |
| | Low | *Reference* | - | *Reference* | - |
| | High | 2.64 (1.19-5.88) | 0.02 | 2.23 (0.96-5.20) | 0.06 |

| WHO grades | | | | | |
|---|---|---|---|---|---|
| | NET G1 | *Reference* | - | *Reference* | - |
| | NET G2 | 1.40 (0.29-6.76) | 0.67 | 1.59 (0.33-7.70) | 0.56 |
| | NET G3 | 9.94 (2.00-49.52) | 0.01 | 7.82 (1.55-39.44) | 0.01 |
| | NEC | 15.55 (3.49-69.37) | <0.001 | 17.09 (3.80-76.81) | <0.001 |

| | | | | | |
|---|---|---|---|---|---|
| ^{∗}uPAR TLR is dichotomized at median (2.47). TLR: target-to-liver ratio. NET: neuroendocrine tumor. NEC: neuroendocrine carcinoma. HR: hazard ratio. CI: confidence interval. | | | | | |

Other cut-offs were evaluated using Cutoff Finder, shown in Figure 3A-B. By using a lower cutoff of TLR (1.32) a smaller group of patients (n=10) with no or a very low risk of death or progression could be identified (Figure 4). Patients with NET G3 and NEC had significantly worse PFS and OS as compared with NET G1, whereas no difference was seen between NET G2 and NET G1 (Table 4 and 5). In multivariate analyses including uPAR expression and WHO classification, both remained significantly associated with PFS, whereas uPAR expression was borderline significantly associated with OS (p=0.06) when controlling for WHO grade.

Also for SUVmax it was possible to establish a cut-off of 2-2.5 where a significant difference in prognosis (hazard ratio (HR) of 3-5 was seen for progression free survival (PFS)) (Figure 5A). Likewise, for SUVmax it was possible to establish (borderline significant) a cut-off of 2.3 where a HR of 4-8 was seen for overall survival (OS) (Figure 5B). However, this only identified 5-6 patients as at low risk. Thus, cut-offs using TLR was superior in relation to establishment of PFS and OS compared to SUVmax.

### Conclusion

uPAR TLR dichotomized at median was significantly associated with PFS and OS where patients with a high uPAR expression had a significantly worse prognosis.

Further, by using a lower cutoff of TLR a smaller group of patients with no or a very low risk of death or progression could be identified.

Importantly Cut-offs established using TLR were superior to cut-offs established using SUVmax. In particular, in relation to companion diagnostics for targeted radiotherapy TLR is relevant, since TLR reflects radiation of tumor vs background, i.e. the effect on tumor relative to irradiation of normal tissue (side effects).

Finally, and as outlined in the summary of the invention, it was also surprisingly found that uPAR-PET was a stronger prognostication tool (higher Hazard Ratios (HR)) (Figure 4A-4B) than commonly used PET modalities for neuroendocrine tumors such as FDG-PET (See Figure 6 - reference figure) and somatostatin receptor imaging, e.g. 64Cu-DOTATATE (see Figure 7 - reference figure). The latter was not significant regarding OS.

### Discussion of data

Our major finding in this study of UPAR PET (exemplified by ⁶⁸Ga-NOTA-AE105 uPAR) was that uPAR expression was seen in the majority of patients with both low and high grade NEN. Furthermore, high uPAR expression was associated with a worse prognosis in regards to both PFS and OS. These findings implies that uPAR could be an attractive target for therapy both due to the availability of the target in patients with NEN and the possibility to specifically target the lesion(s) associated with a poorer prognosis for the patient.

The role of uPA and uPAR in cancer has been extensively investigated in the last decades and it is well established that higher uPAR expression is associated with tumor growth, invasiveness and metastatic spread, although this has not been thoroughly investigated in patients with NEN (18). Accordingly, several therapies targeting uPA and uPAR are undergoing investigation, e.g. an uPAR antibody (huATN-658)(*19*) and a serine protease inhibitor targeting uPA (upamostat)(20). However, none of these therapies have yet been approved for clinical use.

Patients with NEN have highly varying aggressiveness of disease. The primary tumor site, presence of metastases and WHO classification are important prognostic markers and used for guiding selection of treatment (21). Our group and others have shown that in addition low somatostatin receptor density as determined by ⁶⁴Cu-DOTATATE PET and high glucose metabolism as determined by ¹⁸F-FDG PET are prognostic factors (2,3,16,22). With the concept of tailored treatments, specific tumor markers are used to guide eligibility for targeted treatments. This concept has seen widespread implementation in the treatment of patients with NEN, where somatostatin receptor imaging as a companion is used for screening for eligibility to somatostatin receptor targeted therapy with ¹⁷⁷Lu-DOTATATE. In a randomized trial of ¹⁷⁷Lu-DOTATATE patients receiving ¹⁷⁷Lu-DOTATATE compared with high dose of cold somatostatin analogue treatment, the first group had significantly fewer deaths, longer PFS and the response rate was 18% (23). One drawback of targeting somatostatin receptor expressing tumors is the fact that lower expression of somatostatin receptors is seen with the less differentiated and more aggressive tumors (24). On the contrary, uPAR expression is particularly seen in lesions showing tumor growth, invasiveness, and metastatic capability. Previously, our group has investigated uPAR expression by immunohistochemical staining of primary tumor or metastasis from patients with NEN G3 (Ki67 >20%) showing uPAR expression in stromal and or tumor cells in 16/21 (76%) patients (25). However, to the best of our knowledge, expression of uPAR in patients with low grade NEN has never been studied in situ but only indirectly by measurement of soluble uPAR (suPAR) in serum (26). suPAR is the cleaved version of the membrane-bound uPAR and may thus be measured as a circulating uPAR biomarker. The study reported elevated levels of suPAR in patients with NEN compared with healthy controls, and elevated levels of suPAR in both patients with low and high-grade disease. However, no association between suPAR levels and overall survival was seen. Contrary to that study, using PET to visualize uPAR expression at the tumor level is a more direct approach making it possible to identify the lesions with the greatest uPAR expression and the location of these lesions. Also, the expression pattern of uPAR has previously been shown to be heterogeneous with uPAR being highly expressed at the margin of the tumor and thus locally promoting tissue invasion and seeding of metastases, a hallmark of cancer (27). In support of this, we found that high uPAR expression on PET was associated with a worse prognosis, both with regards to PFS and OS. In line with our previous observations in patients with NEC and the data on suPAR in NEN, we found uPAR expression to be present in both low and high grade NEN. Hence, uPAR targeted treatment may be relevant in patients with NEN of all grades. Our observations on uPAR expression should be viewed in the light that the patients included in this study mainly had small intestinal or pancreatic primary tumors with metastatic disease and were previously treated.

A potential innovative perspective is to combine uPAR PET imaging and uPAR-PRRT as a theranostics pair, hence using uPAR PET to assess eligibility to uPAR targeted therapy. We have previously shown a high efficacy of uPAR PRRT in preclinical models of human prostate and colorectal cancer (*11*,*12*), however further studies are warranted to assess the use of uPAR PRRT within NEN. However, the first step towards uPAR-PRRT for NENs was to provide evidence for a high and specific uptake of our uPAR-targeting radioligand and prognostic implications in NEN as done in the present study.

### Summary of data

uPAR expression assessed by ⁶⁸Ga-NOTA-AE105 PET is seen in the majority of patients with both low and high grade NEN and high uPAR expression is associated with a worse prognosis with regards to both PFS and OS. Collectively, this points to uPAR as a relevant target to pursue for risk stratification and possibly also for targeted therapy in patients with NEN.

### References

1. Klimstra DS, Kloppel G, La Rosa S, Rindi G. In: WHO Classification of Tumours Editorial Board. Digestive System Tumours. 5th ed. Lyon, France: International Agency for Research on Cancer; 2019:16-19.
2. Carlsen EA, Johnbeck CB, Loft M, et al. Semi-automatic tumor delineation for evaluation of (64)Cu-DOTATATE PET/CT in patients with neuroendocrine neoplasms: prognostication based on lowest lesion uptake and total tumor volume. J Nucl Med. February 26, 2021 [Epub ahead of print].
3. Binderup T, Knigge U, Johnbeck CB, et al. (18)F-FDG PET is superior to WHO grading as a prognostic tool in neuroendocrine neoplasms and useful in guiding PRRT: a prospective 10-year follow-up study. J Nucl Med. 2021;62:808-815.
4. Mahmood N, Rabbani SA. Fibrinolytic system and cancer: diagnostic and therapeutic applications. Int J Mol Sci. 2021;22:4358.
5. Ahn SB, Mohamedali A, Pascovici D, et al. Proteomics reveals cell-surface urokinase plasminogen activator receptor expression impacts most hallmarks of cancer. Proteomics. 2019;19:e1900026.
6. Mahmood N, Mihalcioiu C, Rabbani SA. Multifaceted role of the urokinase-type plasminogen activator (uPA) and its receptor (uPAR): diagnostic, prognostic, and therapeutic applications. Front Oncol. 2018;8:24.
7. Skovgaard D, Persson M, Brandt-Larsen M, et al. Safety, dosimetry, and tumor detection ability of (68)Ga-NOTA-AE105: first-in-human study of a novel radioligand for uPAR PET imaging. J Nucl Med. 2017;58:379-386.
8. Persson M, Madsen J, Ostergaard S, Ploug M, Kjaer A. 68Ga-labeling and in vivo evaluation of a uPAR binding DOTA- and NODAGA-conjugated peptide for PET imaging of invasive cancers. Nucl Med Biol. 2012;39:560-569.
9. Kriegbaum MC, Persson M, Haldager L, et al. Rational targeting of the urokinase receptor (uPAR): development of antagonists and non-invasive imaging probes. Curr Drug Targets. 2011;12:1711-1728.
10. Fosbol MO, Kurbegovic S, Johannesen HH, et al. Urokinase-type plasminogen activator receptor (uPAR) PET/MRI of prostate cancer for noninvasive evaluation of aggressiveness: comparison with Gleason score in a prospective phase 2 clinical trial. J Nucl Med. 2021;62:354-359.
11. Persson M, Rasmussen P, Madsen J, Ploug M, Kjaer A. New peptide receptor radionuclide therapy of invasive cancer cells: in vivo studies using 177Lu-DOTA-AE105 targeting uPAR in human colorectal cancer xenografts. Nucl Med Biol. 2012;39:962-969.
12. Persson M, Juhl K, Rasmussen P, et al. uPAR targeted radionuclide therapy with (177)Lu-DOTA-AE105 inhibits dissemination of metastatic prostate cancer. Mol Pharm. 2014; 11:2796-2806.
13. Knigge U, Capdevila J, Bartsch DK, et al. ENETS consensus recommendations for the standards of care in neuroendocrine neoplasms: follow-up and documentation. Neuroendocrinology. 2017;105:310-319.
14. Eisenhauer EA, Therasse P, Bogaerts J, et al. New response evaluation criteria in solid tumours: revised RECIST guideline (version 1.1). Eur J Cancer. 2009;45:228-247.
15. Johnbeck CB, Knigge U, Langer SW, et al. Prognostic value of 18F-FLT PET in patients with neuroendocrine neoplasms: a prospective head-to-head comparison with 18F-FDG PET and Ki-67 in 100 patients. J Nucl Med. 2016;57:1851-1857.
16. Binderup T, Knigge U, Loft A, Federspiel B, Kjaer A. 18F-fluorodeoxyglucose positron emission tomography predicts survival of patients with neuroendocrine tumors. Clin Cancer Res. 2010;16:978-985.
17. Budczies J, Klauschen F, Sinn BV, et al. Cutoff Finder: a comprehensive and straightforward web application enabling rapid biomarker cutoff optimization. PLoS One. 2012;7:e51862.
18. Li Santi A, Napolitano F, Montuori N, Ragno P. The urokinase receptor: a multifunctional receptor in cancer cell biology. Therapeutic implications. Int J Mol Sci. 2021;22:4111.
19. Mahmood N, Arakelian A, Khan HA, Tanvir I, Mazar AP, Rabbani SA. uPAR antibody (huATN-658) and Zometa reduce breast cancer growth and skeletal lesions. Bone Res. 2020;8:18.
20. Heinemann V, Ebert MP, Laubender RP, Bevan P, Mala C, Boeck S. Phase II randomised proof-of-concept study of the urokinase inhibitor upamostat (WX-671) in combination with gemcitabine compared with gemcitabine alone in patients with non-resectable, locally advanced pancreatic cancer. Br J Cancer. 2013;108:766-770.
21. Janson ET, Knigge U, Dam G, et al. Nordic guidelines 2021 for diagnosis and treatment of gastroenteropancreatic neuroendocrine neoplasms. Acta Oncol. 2021;60:931-941.
22. Carlsen EA, Johnbeck CB, Binderup T, et al. (64)Cu-DOTATATE PET/CT and prediction of overall and progression-free survival in patients with neuroendocrine neoplasms. J Nucl Med. 2020;61:1491-1497.
23. Strosberg J, El-Haddad G, Wolin E, et al. Phase 3 trial of (177)Lu-Dotatate for midgut neuroendocrine tumors. N Engl J Med. 2017;376:125-135.
24. Sorbye H, Kong G, Grozinsky-Glasberg S. PRRT in high-grade gastroenteropancreatic neuroendocrine neoplasms (WHO G3). Endocr Relat Cancer. 2020;27:R67-R77.
25. Olsen IH, Langer SW, Federspiel BH, et al. (68)Ga-DOTATOC PET and gene expression profile in patients with neuroendocrine carcinomas: strong correlation between PET tracer uptake and gene expression of somatostatin receptor subtype 2. Am J Nucl Med Mol Imaging. 2016;6:59-72.
26. Ozdirik B, Stueven A, Knorr J, et al. Soluble urokinase plasminogen activator receptor (suPAR) concentrations are elevated in patients with neuroendocrine malignancies. J Clin Med. 2020;9:1647.
27. Hanahan D, Weinberg RA. Hallmarks of cancer: the next generation. Cell. 2011;144:646-674.
28. Persson M, Skovgaard D, Brandt-Larsen M, et al. First-in-human uPAR PET: Imaging of cancer aggressiveness. Theranostics. 2015;5:1303-1316.

## Claims

1. A positron-emitting imaging agent for use in the prognostication of a neuroendocrine neoplasm (NEN) patient by PET imaging of the cancer,
wherein said imaging agent comprises a uPAR binding peptide coupled via the chelating agent NOTA to the radionuclide 68Ga; and
wherein the uPAR binding peptide is (D-Asp)-([beta]-cyclohexyl-L-alanine)-(Phe)-(D-Ser)-(D-Arg)-(Tyr)-(Leu)-(Trp)-(Ser) or a uPAR binding variant thereof.

2. The imaging agent for use according to claim 1, wherein the uPAR binding variant is selected from the group consisting of
• (D-Asp)-([beta]-cyclohexyl-L-alanine)-(Phe)-(D-Ser)-(D-Arg)-(Tyr)-(Leu)-(Trp)-(Ser),
• (Ser)-(Leu)-([beta]-cyclohexyl-L-alanine)-(Phe)-(D-Ser)-(Gln)-(Tyr)(Leu)-(Trp)-(Ser),
• (D-Glu)-([beta]-cyclohexyl-L-alanine)-(Phe)-(D-Ser)-(D-Tyr)-(Tyr)-(Leu)-(Trp)-(Ser),
• (Asp)-([beta]-cyclohexyl-L-alanine)-(Phe)-(D-Ser)-(D-Arg)-(Tyr)-(Leu)-(Trp)-(Ser),
• (Asp)-([beta]-cyclohexyl-L-alanine)-(Phe)-(Ser)-(D-Arg)-(Tyr)-Leu)-(Trp)-(Ser),
• (D-Asp)-([beta]-cyclohexyl-L-alanine)-(Phe)-(Ser)-(D-Arg)-(Tyr)-Leu)-(Trp)-(Ser),
• (D-Thr)-([beta]-cyclohexyl-L-alanine)-(Phe)-(D-Ser)-(D-Arg)-(Tyr)-(Leu)-(Trp)-(Ser),
• (D-Asp)-([beta]-cyclohexyl-L-alanine)-(Phe)-(D-Ser)-(D-Arg)-(Tyr)-(Leu)-([beta]-2-naphthyl-L-alanine)-(Ser),
• (Asp)-([beta]-cyclohexyl-L-alanine)-(Phe)-(D-Ser)-(Arg)-(Tyr)-(Leu)-(Trp)-(Ser),
• (Asp)-([beta]-cyclohexyl-L-alanine)-(Phe)-(D-Ser)-(D-Arg)-(Tyr)-(Leu)([beta]-1-naphthyl-L-alanine)-(Ser),
• (D-Glu)-([beta]-cyclohexyl-L-alanine)-(Phe)-(D-Ser)-(Tyr)-(Tyr)-(Leu)-(Trp)-(Ser),
• (Asp)-([beta]-cyclohexyl-L-alanine)-(Phe)-(D-Ser)-(D-Arg)-(Leu)-(Leu)-(Trp)-(D-His),
• (Asp)-([beta]-cyclohexyl-L-alanine)-(Phe)-(D-Ser)-(D-Arg)-([beta]-cyclohexyl-L-alanine)-(Leu)-(Trp)-(Ile),
• (Asp)-([beta]-cyclohexyl-L-alanine)-(Phe)-(D-Ser)-(D-Arg)-(Tyr)-(Leu)([beta]-1-naphthyl-L-alanine)-(D-His),
• (Asp)-([beta]-cyclohexyl-L-alanine)-(Phe)-(D-Ser)-(D-Arg)-(N-(2,3-dimethoxybenzyl)glycine)-(D-Phe)-(N-(3-indolylethyl)glycine)-(N-(2-methoxyethyl)glycine),
• (Asp)-([beta]-cyclohexyl-L-alanine)-(Phe)-(D-Ser)-(D-Arg)-(N-(2,3-dimethoxybenzyl)glycine)-(D-Phe)-(N-benzylglycine)-(N-(2[beta]thoxyethyl)glycine),
• (Asp)-([beta]-cyclohexyl-L-alanine)-(Phe)-(D-Ser)-(D-Arg)-(N-(2,3-dimethoxybenzyl)glycine)-(D-Phe)-(N-(methylnaphthalyl)glycine)-(N-(2-methoxyethyl)glycine), and
• (Asp)-([beta]-cyclohexyl-L-alanine)-(Phe)-(D-Ser)-(D-Arg)-(N-(2,3-dimethoxybenzyl)glycine)-(D-Phe)-(N-(2,3-dimethoxybenzyl)glycine)-(Ile).

3. The imaging agent for use according to claim 1 or 2, wherein the peptide is (D-Asp)-([beta]-cyclohexyl-L-alanine)-(Phe)-(D-Ser)-(D-Arg)-(Tyr)-(Leu)-(Trp)-(Ser).

4. The imaging agent for use according to any of the preceding claims, having the formula

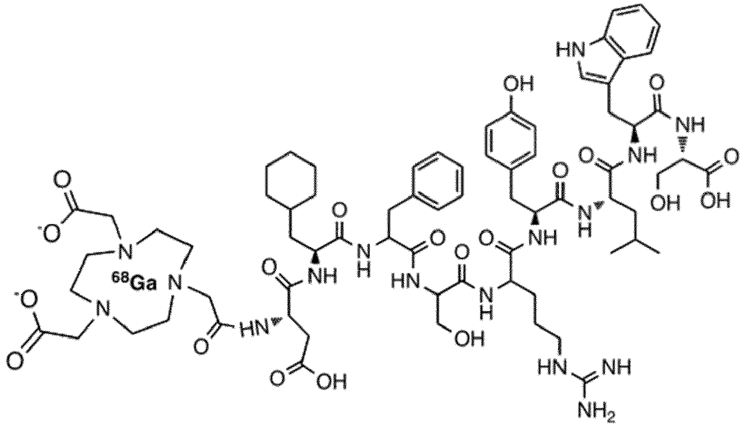

5. The imaging agent for use according to any of the preceding claims,
wherein the prognosis is prognosis of relapse-free survival (RFS) and/or overall survival (OS).

6. The imaging agent for use according to any of the preceding claims,
wherein the imaging agent is to be administered in a dose of 10-500 MBq followed by PET scanning 10 min to 24 hours after the imaging agent has been administered, and quantification through SUVmax and/or SUVmean.

7. The imaging agent for use according to any of the preceding claims,
∘ wherein a SUVmax and/or SUVmean and/or target-to liver ratio (TTR) quantification level above a threshold level is indicative of a poor Relapse-free survival (RFS) prognosis and/or poor overall survival (OS) prognosis; and
∘ wherein a SUVmax and/or SUVmean and/or target-to liver ratio (TTR) quantification level equal to or below a threshold level is indicative of good Relapse-free survival (RFS) prognosis and/or good overall survival (OS) prognosis.

8. The imaging agent for use according to claim 6 or 7, wherein said target-to liver ratio (TLR) threshold level is in the range 1.1-4, such as in the range 1.3-4, such as in the range 2-4 or 2-3.

9. The imaging agent for use according to any of claims 6-8, wherein said target-to liver ratio (TLR) threshold level is in the range 1.1-2, such as in the range 1.1-1.5, such as in the range 1.2-1.4.

10. A method of in vivo imaging by PET imaging, for assessing the prognosis of a Neuroendocrine neoplasm (NEN) in a patient, said method comprising:
a) provision of a subject to which a imaging agent as defined in any of claims 1-9 has been previously administered;
b) detecting by in vivo PET imaging the radioactive emissions from the radioisotope of the administered imaging agent of step a);
c) generating an image representative of the location and/or amount of said radioactive emissions;
d) determining the distribution and extent of uPAR expression in said subject, wherein said expression is correlated with said signals emitted by said in vivo imaging agent; and
e) comparing the determined distribution and extent of uPAR expression to a threshold level;
▪ wherein a SUVmax and/or SUVmean and/or TLR quantification level above a threshold level is indicative of a poor Relapse-free survival (RFS) prognosis and/or poor overall survival (OS) prognosis; and
▪ wherein a SUVmax and/or SUVmean and/or TLR quantification level equal to or below a threshold level is indicative of good Relapse-free survival (RFS) prognosis and/or good overall survival (OS) prognosis.

11. The method according to claim 10, wherein if a good Relapse-free survival (RFS) prognosis or good overall survival (OS) prognosis is indicated, said subject will be scheduled for a de-escalated treatment regimen to avoid unnecessary toxicity and/or for a risk stratified follow-up schedule;
or
wherein if a poor Relapse-free survival (RFS) prognosis or poor overall survival (OS) prognosis is indicated, said subject is scheduled for radiotherapy, such as wherein said radiotherapy is with a radiopharmaceutical comprises a radionuclide and a uPAR binding peptide.

12. A positron-emitting imaging agent for use as companion diagnostic of a neuroendocrine neoplasm (NEN) patient by PET imaging of the cancer,
wherein said imaging agent comprises a uPAR binding peptide coupled via the chelating agent NOTA or DOTA to the radionuclide 68Ga or 64Cu; and
wherein the uPAR binding peptide is (D-Asp)-([beta]-cyclohexyl-L-alanine)-(Phe)-(D-Ser)-(D-Arg)-(Tyr)-(Leu)-(Trp)-(Ser) or a uPAR binding variant thereof;
wherein
∘ a SUVmax and/or SUVmean and/or target-to liver ratio (TLR) quantification level above a threshold level is indicative of that a uPAR binding drug, such as a uPAR binding radiopharmaceutical will be effective against said NEN; and
∘ a SUVmax and/or SUVmean and/or target-to liver ratio (TLR) quantification level equal to or below a threshold level is indicative of that a uPAR drug, such as a uPAR binding radiopharmaceutical will not be effective against said NEN.

13. A composition comprising a radiopharmaceutical for use in the treatment or alleviation of a neuroendocrine neoplasm (NEN) in a subject;
wherein said radiopharmaceutical comprises a radionuclide and a uPAR binding peptide; and
wherein the uPAR binding peptide is (D-Asp)-([beta]-cyclohexyl-L-alanine)-(Phe)-(D-Ser)-(D-Arg)-(Tyr)-(Leu)-(Trp)-(Ser) or a uPAR binding variant thereof; and
wherein said subject has been diagnosed with a uPAR expressing neuroendocrine neoplasm (NEN); and wherein said uPAR expression is above a predetermined threshold level,
wherein said threshold level is determined using a positron-emitting imaging agent as defined in any of claims 1-9.

14. The composition for use according to claim 11, wherein the radiopharmaceutical is 177Lu-DOTA-AE105.

15. The composition for use according to any of claims 13-14, wherein said target-to liver ratio (TLR) threshold level is in the range 1.1-4, such as in the range 1.3-4, such as in the range 2-4 or 2-3, or wherein said target-to liver ratio (TLR) threshold level is in the range 1.1-2, such as in the range 1.1-1.5, such as in the range 1.2-1.4.
